# EUROPEAN PATENT APPLICATION

(11) **EP 3 604 477 A1**
(43) Date of publication of application: **05.02.2020**
(21) Application number: 18186366.3
(22) Date of filing: 30.07.2018
(51) Int. Cl.: C09K 11/06, C07D 209/00, H01L 51/00

(54) **POLYCYCLIC COMPOUND, ORGANIC ELECTROLUMINESCENCE DEVICE, AND ELECTRONIC DEVICE**

(71) Applicant: Idemitsu Kosan Co., Ltd., Chiyoda-ku, Tokyo 100-8321 (JP)
(72) Inventor: Sustac-Roman, Daniela, 4055 Basel (CH); Nishimae, Yuichi, 4058 Basel (CH); Chebotareva, Natalia, 68220 Hagenthal le Bas (FR)
(74) Representative: Hollah, Dorothee

(57) **Abstract**

Specific polycyclic compounds of the general formula (I) and a process for their preparation, a material for an organic electroluminescence device comprising said compound, an organic electroluminescence device comprising said compound, an electronic equipment comprising said organic electroluminescence device, and the use of compounds according to general formula (I) in an organic electroluminescence device.

## Description

The present invention relates to specific polycyclic compounds, organic electroluminescence devices (organic EL devices) and electronic devices comprising the same.

JP2012-140365 A relates to polycyclic compounds of formulae (1) to (6) and to an organic EL element comprising the same.

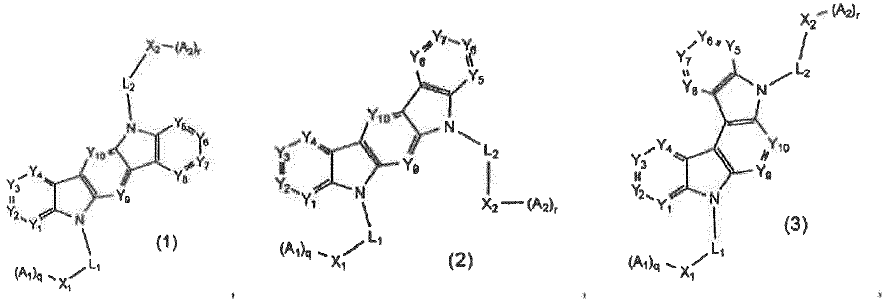

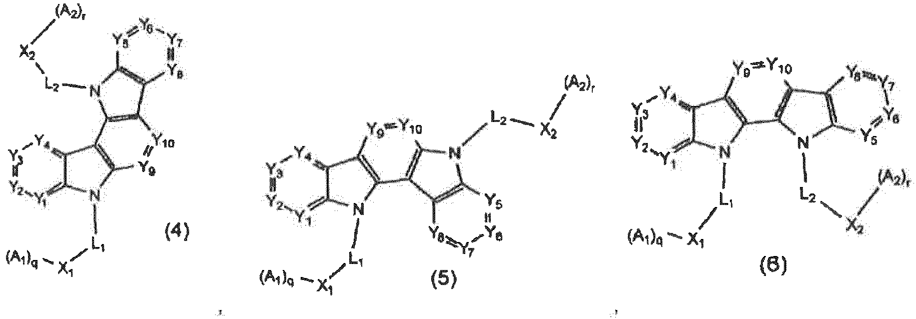

WO2010/107244 A2 relates to organic electroluminescent compounds and organic electroluminescent devices comprising the same. The organic electroluminescent compounds are employed as electroluminescent material and the organic electroluminescent devices using them as a host. The polycyclic compounds have the chemical formulae 1 to 5:

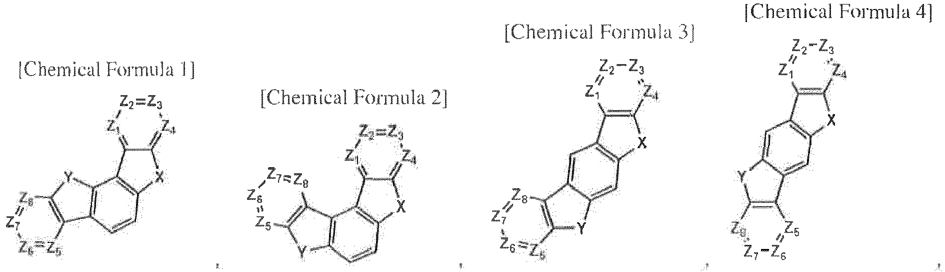

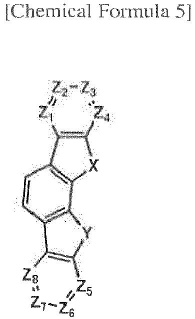

KR101627750 A relates to a compound, an organic light emitting device comprising the same, and a display device comprising the organic light emitting device. The compound has the following formula:

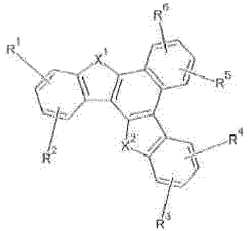

While the materials presented in the relevant art above demonstrate satisfactory light emitting properties in organic EL devices, there still remains a need to further improve their performances such as achieving lower driving voltages, better external quantum efficiencies and/or improved device lifetimes.

Accordingly, it is an object of the present invention, with respect to the aforementioned related art, to provide further materials suitable for use in organic EL devices and further applications in organic electronics. More particularly, it should be possible to provide charge transport materials, e.g. electron transport materials, and/or charge blocker materials, e.g. hole blocker materials, and/or host (= matrix) materials for use in organic EL devices. The materials should be suitable especially for organic EL devices which comprise at least one emitter, which is a phosphorescence emitter and/or a fluorescence emitter.

Furthermore, the materials should be suitable for providing organic EL devices which ensure good performance of the organic EL devices, especially good lifetimes, good external quantum efficiencies and/or low driving voltages.

Said object is achieved by a polycyclic compound represented by formula (I): 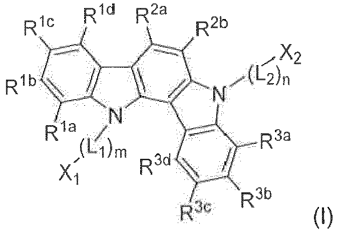 wherein,
R^{1a}, R^{1b}, R^{1c}, R^{1d}, R^{2a}, R^{2b}, R^{3a}, R^{3b}, R^{3c} and R^{3d} are independently of each other a hydrogen atom, a substituted or unsubstituted alkyl or cycloalkyl group having 1 to 20 carbon atoms, a substituted or unsubstituted alkoxy or cycloalkoxy group having 1 to 20 carbon atoms, a substituted or unsubstituted alkenyl group having 2 to 20 carbon atoms, a substituted or unsubstituted alkynyl group having 2 to 20 carbon atoms, a substituted or unsubstituted aryl group having 6 to 30 ring carbon atoms, a substituted or unsubstituted heteroaryl group having 5 to 30 ring carbon atoms,
wherein
at least two adjacent groups R^{1a} and R^{1b}, R^{1b} and R^{1c} or R^{1c} and R^{1d}, R^{2a} and R^{2b}, and/or R^{3a} and R^{3b}, R^{3b} and R^{3c} or R^{3c} and R^{3d} are linked together to form a ring;
L₁ and L₂ are each independently a direct bond, a substituted or unsubstituted arylene group having 6 to 30 ring carbon atoms, a substituted or unsubstituted heteroarylene group having 5 to 30 ring carbon atoms;
X₁ and X₂ are each independently a substituted or unsubstituted aryl group having 6 to 30 ring carbon atoms, a substituted or unsubstituted O- or S-containing heteroaryl group having 5 to 30 ring carbon atoms;
m and n are independently of each other 1 or 2,
wherein at least one of X₁ and X₂ represents a substituted or unsubstituted fluoranthene unit.

The compounds of formula (I) according to the present invention are specifically characterized by a combination of the following features:
- at least one fluoranthene unit substituted at one of the N atoms in the base skeleton of the compounds of formula (I);
- at least six condensed rings in the base skeleton of the compounds of formula (I);
- a specific arrangement of the N atoms in the two five membered rings mandatorily present in the compounds of formula (I).

Only by a combination of the specific features of the compounds of formula (I) according to the present invention, superior lifetimes, external quantum efficiencies and/or driving voltages are achieved.

The compounds of formula (I) of the present invention may be used as a material, especially host, charge transport or charge blocking material being highly suitable in organic EL devices. Moreover, a balanced charge transport and/or charge blocking in devices is achieved, especially resulting in good lifetimes, good external quantum efficiencies and/or low driving voltages.

The compounds of the present invention are preferably used in organic EL devices, for example, in organic light-emitting diodes (OLEDs).

Accordingly, a further subject of the present invention is directed to an organic EL device, such as an organic light-emitting diode (OLED), comprising a compound according to the present invention.

The compounds of formula (I) can in principal be used in any layer of an organic EL device, but are preferably used as host, charge transport, especially electron transport, and/or charge blocking, especially hole blocking, material. More preferably, the compounds of formula (I) are used as host material, hole blocking material and/or electron transport material for phosphorescence or fluorescence emitters, most preferably, the compounds of formula (I) are used as host material for phosphorescence or fluorescence emitters, even more most preferably, the compounds of formula (I) are used as host material for phosphorescence emitters.

Hence, a further subject of the present invention is directed to an emitting layer, comprising a compound of formula (I) according to the present invention. In said embodiment a compound of formula (I) is preferably used as host material alone or together with one or more, preferably one, further host materials (co-host material). More preferably, a combination of a compound of formula (I) as host material together with a phosphorescent emitter is used.

The terms hydrogen atom, substituted or unsubstituted alkyl or cycloalkyl group having 1 to 20 carbon atoms, substituted or unsubstituted alkoxy or cycloalkoxy group having 1 to 20 carbon atoms, substituted or unsubstituted alkenyl group having 2 to 20 carbon atoms, substituted or unsubstituted alkynyl group having 2 to 20 carbon atoms, substituted or unsubstituted aryl group having 6 to 30 ring carbon atoms, substituted or unsubstituted heteroaryl group having 3 to 30 ring carbon atoms, substituted or unsubstituted arylene group having 6 to 30 ring carbon atoms, substituted or unsubstituted heteroarylene group having 3 to 30 ring carbon atoms, substituted or unsubstituted O- or S-containing heteroarylene group having 4 to 30 ring carbon atoms, substituted or unsubstituted aralkyl group having 7 to 24 carbon atoms, halogen atom, silyl groups, haloalkyl group having 1 to 20 carbon atoms, haloalkoxy group having 1 to 20 carbon atoms, aryloxy group having 6 to 30 ring carbon atoms, alkylthio group having 1 to 20 carbon atoms, arylthio group having 6 to 30 ring carbon atoms, an alkylamino group having 1 to 20 carbon atoms, an arylamino group having 6 to 30 carbon atoms, a carboxyalkyl group having 1 to 20 carbon atoms, a carboxamidalkyl group having 1 to 20 carbon atoms, a carboxyaryl group having 6 to 30 carbon atoms, a carboxamidaryl group having 6 to 30 carbon atoms
are known in the art and generally have the following meaning, if said groups are not further specified in specific embodiments mentioned below:
The substituted or unsubstituted aryl group having 6 to 30 ring carbon atoms, preferably 6 to 18 ring carbon atoms, may be a non-condensed aromatic hydrocarbon group or a condensed aromatic hydrocarbon group. Specific examples thereof include phenyl group, naphthyl group, phenanthryl group, biphenyl group, terphenyl group, quaterphenyl group, fluoranthenyl group, triphenylenyl group, phenanthrenyl group, fluorenyl group, anthracenyl, chrysenyl, spirofluorenyl group, 9,9-diphenylfluorenyl group, 9,9'-spirobi[9H-fluorene]-2-yl group, 9,9-dimethylfluorenyl group, benzo[c]phenanthrenyl group, benzo[a]triphenylenyl group, naphtho[1,2-c]phenanthrenyl group, naphtho[1,2-a]triphenylenyl group, dibenzo[a,c]triphenylenyl group, benzo[a]fluoranthenyl group, benzo[j]fluoranthenyl group, benzo[k]fluoranthenyl group and benzo[b]fluoranthenyl group, with phenyl group, naphthyl group, biphenyl group, terphenyl group, phenanthryl group, triphenylenyl group, fluorenyl group, spirobifluorenyl group, and fluoranthenyl group being preferred, and phenyl group, 1-naphthyl group, 2-naphthyl group, biphenyl-2-yl group, biphenyl-3-yl group, biphenyl-4-yl group, phenanthrene-9-yl group, phenanthrene-3-yl group, phenanthrene-2-yl group, triphenylene-2-yl group, 9,9-dimethylfluorene-2-yl group, fluoranthene-3-yl group, fluoranthene-2-yl group, fluoranthene-8-yl group being more preferred.

The substituted or unsubstituted heteroaryl group having 3 to 30 ring carbon atoms, preferably 5 to 18 ring atoms, may be a non-condensed heterocyclic group or a condensed heterocyclic group. Specific examples thereof include the residues of pyrrole ring, isoindole ring, benzofuran ring, isobenzofuran ring, benzothiophene, dibenzothiophene ring, isoquinoline ring, quinoxaline ring, quinazoline, phenanthridine ring, phenanthroline ring, pyridine ring, pyrazine ring, pyrimidine ring, pyridazine ring, triazine ring, indole ring, quinoline ring, acridine ring, pyrrolidine ring, dioxane ring, piperidine ring, morpholine ring, piperazine ring, carbazole ring, furan ring, thiophene ring, oxazole ring, oxadiazole ring, benzoxazole ring, thiazole ring, thiadiazole ring, benzothiazole ring, triazole ring, imidazole ring, benzimidazole ring, pyran ring, dibenzofuran ring and benzo[c]dibenzofuran ring, and the residues of compounds of these rings, with the residues of dibenzofuran ring, carbazole ring, dibenzothiophene ring, and compounds of these rings being preferred, and the residues of dibenzofuran-2-yl group, dibenzofuran-4-yl group, dibenzofuran-1-yl group, dibenzofuran-3-yl group,9-phenylcarbazole-3-yl group, 9-phenylcarbazole-2-yl group, 9-phenylcarbazole-4-yl group, dibenzothiophene-2-yl group, and dibenzothiophene-4-yl group, dibenzothiophene-1-yl group, and dibenzothiophene-3-yl group being more preferred.

The substituted or unsubstituted O- or S-containing heteroaryl group having 4 to 30 ring carbon atoms is defined as the substituted or unsubstituted heteroaryl group having 4 to 30 ring carbon atoms mentioned above, comprising as heteroatoms O or S, and may be a non-condensed O- or S-containing heteroaryl group or a condensed O- or S-containing heteroaryl group.

Examples of the substituted or unsubstituted alkyl group having 1 to 20 carbon atoms include methyl group, ethyl group, n-propyl group, isopropyl group, n-butyl group, s-butyl group, isobutyl group, t-butyl group, n-pentyl group, n-hexyl group, n-heptyl group, n-octyl group, n-nonyl group, n-decyl group, n-undecyl group, n-dodecyl group, n-tridecyl group, n-tetradecyl group, n-pentadecyl group, n-hexadecyl group, n-heptadecyl group, n-octadecyl group, neopentyl group, 1-methylpentyl group, with methyl group, ethyl group, n-propyl group, isopropyl group, n-butyl group, s-butyl group, isobutyl group, t-butyl group being preferred.

Examples of the substituted or unsubstituted cycloalkyl group are substituted or unsubstituted cycloalkyl groups having 3 to 20 carbon atoms, including cyclopropyl group, cyclobutyl group, cyclopentyl group, cyclohexyl group, cyclooctyl group, and adamantyl group, with cyclopentyl group, and cyclohexyl group being preferred.

The substituted or unsubstituted arylene group having 6 to 30 ring carbon atoms is a non-condensed divalent aromatic hydrocarbon group or a condensed divalent aromatic hydrocarbon group. Specific examples thereof include phenylene group, naphthylene group, phenanthrylene group, biphenyl-diyl group, terphenyl-diyl group, quaterphenyl-diyl group, fluoranthen-diyl group, triphenylenylene-diyl group, phenanthrene-diyl group, fluorene-diyl group, spirofluorene-diyl group, 9,9-diphenylfluorene-diyl group, 9,9'-spirobi[9H-fluorene]-2-diyl group, 9,9-dimethylfluorene-diyl group, benzo[c]phenanthrene-diyl group, benzo[a]triphenylene-diyl group, naphtho[1,2-c]phenanthrene-diyl group, naphtho[1,2-a]triphenylenylene-diyl group, dibenzo[a,c]triphenylenylene-diyl group, benzo[a]fluoranthene-diyl group, benzo[j]fluoranthene-diyl group, benzo[k]fluoranthene-diyl group,and benzo[b]fluoranthene-diyl group, with phenylene group, naphthylene group, biphenyl-diyl group, terphenyl-diyl group, phenanthryl-diyl group, triphenylenylen-diyl group, fluorene-diyl group, spirobifluorene-diyl group, and fluoranthene-diyl group being preferred, and 1,2-phenylene group, 1,3-phenylene group, 1,4-phenylene group, 1,4-naphthylene group, 1,8-naphthylene group, 2,6-naphthylene group, 2,7-naphthylene group, biphenyl-2,2'-diyl group, biphenyl-2,3'-diyl group, biphenyl-2,4'-diyl group, biphenyl-2,5'-diyl group, biphenyl-2,6'-diyl group, biphenyl-3,3'-diyl group, biphenyl-3,4'-diyl group, biphenyl-3,5'-diyl group, biphenyl-3,6'-diyl group, biphenyl-4,4'-diyl group, biphenyl-4,5'-diyl group, biphenyl-4,6'-diyl group, biphenyl-5,5'-diyl group, biphenyl-5,6'-diyl group, biphenyl-6,6'-diyl group, phenanthrene-9,10-diyl group, phenanthrene-2,3-diyl group, phenanthrene-2,7-diyl group, phenanthrene-2,8-diyl group, phenanthrene-2,6-diyl group, phenanthrene-2,9-diyl group, phenanthrene-2,10-diyl group, phenanthrene-3,9-diyl group, phenanthrene-3,10-diyl group, triphenylene-2,3-diyl group, triphenylene-2,5-diyl group, triphenylene-2,6-diyl group, triphenylene-2,7-diyl group, triphenylene-2,8-diyl group, 9,9-dimethylfluorene-2,7-diyl group, 9,9-dimethylfluorene-3,7-diyl group, 9,9-dimethylfluorene-1,4-diyl group, fluoranthene-3,9-diyl group, fluoranthene-3,8-diyl group, fluoranthene-3,4-diyl group, fluoranthene-3,5-diyl group, fluoranthene-3,6-diyl group, fluoranthene-2,9-diyl group, fluoranthene-2,8-diyl group, fluoranthene-2,4-diyl group, fluoranthene-2,5-diyl group, fluoranthene-2,6-diyl group, fluoranthene-1,9-diyl group, fluoranthene-1,8-diyl group, fluoranthene-1,4-diyl group, fluoranthene-1,5-diyl group, fluoranthene-1,6-diyl group being more preferred.

The substituted or unsubstituted heteroarylene group having 3 to 30 ring carbon atoms is a non-condensed heterocyclic group or a condensed heterocyclic group. Specific examples thereof include the divalent residues of pyrrole ring, isoindole ring, benzofuran ring, isobenzofuran ring, dibenzothiophene ring, isoquinoline ring, quinoxaline ring, quinazoline, phenanthridine ring, phenanthroline ring, pyridine ring, pyrazine ring, pyrimidine ring, pyridazine ring, triazine ring, indole ring, quinoline ring, acridine ring, pyrrolidine ring, dioxane ring, piperidine ring, morpholine ring, piperazine ring, carbazole ring, furan ring, thiophene ring, oxazole ring, oxadiazole ring, benzoxazole ring, thiazole ring, thiadiazole ring, benzothiazole ring, triazole ring, imidazole ring, benzimidazole ring, pyran ring, dibenzofuran ring, and benzo[c]dibenzofuran ring, and the divalent residues of compounds of these rings, with the divalent residues of dibenzofuran ring, carbazole ring, dibenzothiophene ring, and compounds of these divalent rings being preferred, and the dibenzofuran-diyl group, 9-phenylcarbazole-diyl group and dibenzothiophene-diyl group being more preferred.

Examples of the substituted or unsubstituted alkoxy group having 1 to 20 carbon atoms include those having an alkyl portion selected from the alkyl groups mentioned above.

Examples of the substituted or unsubstituted cycloalkoxy group are substituted or unsubstituted cycloalkoxy groups having 2 to 20 carbon atoms, including those having a cycloalkyl portion selected from the alkyl groups mentioned above.

The substituted or unsubstituted alkenyl group having 2 to 20 carbon atoms is a group having a double bond in the above alkyl group and has preferably 2 to 10 carbon atoms. An alkenyl group is more preferably a vinyl group.

The substituted or unsubstituted alkynyl group having 2 to 20 carbon atoms is a group having a triple bond in the above alkyl group and has preferably 2 to 10 carbon atoms. The alkynyl group is more preferably an ethynyl group.

Examples of a substituted or unsubstituted aralkyl group having 7 to 24 carbon atoms, preferably 7 to 20 carbon atoms, include benzyl group, 2-phenylpropane-2-yl group, 1-phenylethyl group, 2-phenylethyl group, 1-phenylisopropyl group, 2-phenylisopropyl group, phenyl-t-butyl group, α-naphthylmethyl group, 1-α-naphthylethyl group, 2-α-naphthylethyl group, 1-α-naphthylisopropyl group, 2-α-naphthylisopropyl group, β-naphthylmethyl group, 1-β-naphthylethyl group, 2-β-naphthylethyl group, 1-β-naphthylisopropyl group, 2-β-naphthylisopropyl group, 1-pyrrolylmethyl group, 2-(1-pyrrolyl)ethyl group, p-methylbenzyl group, m-methylbenzyl group, o-methylbenzyl group, p-chlorobenzyl group, m-chlorobenzyl group, o-chlorobenzyl group, p-bromobenzyl group, m-bromobenzyl group, o-bromobenzyl group, p-iodobenzyl group, m-iodobenzyl group, o-iodobenzyl group, p-hydroxybenzyl group, m-hydroxybenzyl group, o-hydroxybenzyl group, p-aminobenzyl group, m-aminobenzyl group, o-aminobenzyl group, p-nitrobenzyl group, m-nitrobenzyl group, o-nitrobenzyl group, p-cyanobenzyl group, m-cyanobenzyl group, o-cyanobenzyl group, 1-hydroxy-2-phenylisopropyl group, and 1-chloro-2-phenylisopropyl group.

Examples of silyl groups include alkylsilyl groups having 1 to 10 carbon atoms, preferably 1 to 5 carbon atoms, including trimethylsilyl group, triethylsilyl group, tributylsilyl group, dimethylethylsilyl group, t-butyldimethylsilyl group, vinyldimethylsilyl group, propyldimethylsilyl group, dimethylisopropylsilyl group, dimethylpropylsilyl group, dimethylbutylsilyl group, dimethyltertiarybutylsilyl group, diethylisopropylsilyl group, and arylsilyl groups having 6 to 30 carbon atoms, preferably 6 to 18 carbon atoms, including phenyldimethylsilyl group, diphenylmethylsilyl group, diphenyltertiarybutylsilyl group, and triphenylsilyl group, with trimethylsilyl group, triethylsilyl group, t-butyldimethylsilyl group, vinyldimethylsilyl group, and propyldimethylsilyl group being preferred.

Examples of halogen atoms include fluorine, chlorine, bromine, and iodine, with fluorine being preferred.

Examples of a haloalkyl group having 1 to 20 carbon atoms include the alkyl groups mentioned above wherein the hydrogen atoms thereof are partly or entirely substituted by halogen atoms.

Examples of a haloalkoxy group having 1 to 20 carbon atoms include the alkoxyl group mentioned above wherein the hydrogen atoms thereof are partly or entirely substituted by halogen atoms.

Examples of an aryloxy group having 6 to 30 ring carbon atoms include those having an aryl portion selected from the aromatic hydrocarbon groups mentioned above.

Examples of an alkylthio group having 1 to 20 carbon atoms include those having an alkyl portion selected from the alkyl groups mentioned above.

Examples of an arylthio group having 6 to 30 ring carbon atoms include those having an aryl portion selected from the aromatic hydrocarbon groups mentioned above.

Examples of an alkylamino group having 1 to 20 ring carbon atoms include those having an alkyl portion selected from the alkyl groups mentioned above.

Examples of an arylamino group having 6 to 30 ring carbon atoms include those having an aryl portion selected from the aromatic hydrocarbon groups mentioned above.

Examples of a carboxyalkyl group having 1 to 20 carbon atoms, preferably 1 to 5 carbon atoms, include those having an alkyl portion selected from the alkyl groups mentioned above.

Examples of a carboxamidalkyl group having 1 to 20 carbon atoms, preferably 1 to 5 carbon atom include those having an alkyl portion selected from the alkyl groups mentioned above.

Examples of a carboxyaryl group having 6 to 30 carbon atoms, preferably 6 to 18 carbon atoms, include those having an aryl portion selected from the aromatic hydrocarbon groups mentioned above.

Examples of a carboxamidaryl group having 6 to 30 carbon atoms, preferably 6 to 18 carbon atoms, include those having an aryl portion selected from the aromatic hydrocarbon groups mentioned above.

Examples of the optional substituent(s) indicated by "substituted or unsubstituted" and "may be substituted" referred to above or hereinafter include a halogen atom (fluorine, chlorine, bromine, iodine), a cyano group, an alkyl group having 1 to 20, preferably 1 to 6 carbon atoms, a cycloalkyl group having 3 to 20, preferably 5 to 12 carbon atoms, an alkoxy group having 1 to 20, preferably 1 to 5 carbon atoms, a haloalkyl group having 1 to 20, preferably 1 to 5 carbon atoms, a haloalkoxyl group having 1 to 20, preferably 1 to 5 carbon atoms, an alkylamino group having 1 to 20 carbon atoms, preferably 1 to 5 carbon atoms, a carboxyalkyl group having 1 to 20 carbon atoms, preferably 1 to 5 carbon atoms, a carboxamidalkyl group having 1 to 20 carbon atoms, preferably 1 to 5 carbon atoms, a silyl group, an aromatic hydrocarbon group having 6 to 30 ring carbon atoms, preferably 6 to 18 ring carbon atoms, an aryloxy group having 6 to 30, preferably 6 to 18 ring carbon atoms, a substituted or unsubstituted aralkyl group having 7 to 24, preferably 7 to 20 carbon atoms, an alkylthio group having 1 to 20, preferably 1 to 5 carbon atoms, an arylthio group having 6 to 30, preferably 6 to 18 ring carbon atoms, an arylamino group having 6 to 30 carbon atoms, preferably 6 to 18 carbon atoms, a carboxyaryl group having 6 to 30 carbon atoms, preferably 6 to 18 carbon atoms, a carboxamidaryl group having 6 to 30 carbon atoms, preferably 6 to 18 carbon atoms, and a heterocyclic group having 5 to 30 ring atoms, preferably 5 to 18 ring atoms.

The optional substituent is preferably a fluorine atom, a cyano group, an alkyl group having 1 to 30 carbon atoms, a silyl group, an aromatic hydrocarbon group having 6 to 30 ring carbon atoms, preferably 6 to 18 ring carbon atoms, and an heterocyclic group having 5 to 30 ring atoms, preferably 5 to 18 ring atoms; more preferably a fluorine atom, a silyl group, a cyano group, a phenyl group, a naphthyl group, a biphenyl group, a terphenyl group, a phenanthryl group, a triphenylenyl group, a fluorenyl group, a spirobifluorenyl group, a fluoranthenyl group, a residue based on a dibenzofuran ring, a residue based on a carbazole ring, a residue based on a dibenzothiophene ring, a methyl group, an ethyl group, an n-propyl group, an isopropyl group, an n-butyl group, an s-butyl group, an isobutyl group, a t-butyl group, a cyclopentyl group, and a cyclohexyl group.

The optional substituents mentioned above may be further substituted by one or more of the optional substituents mentioned above.

The number of the optional substituents depends on the group which is substituted by said substituent(s). Preferred are 1, 2, 3 or 4 optional substituents, more preferred are 1, 2 or 3 optional substituents, most preferred are 1 or 2 optional substituents. In a further preferred embodiment, the groups mentioned above are unsubstituted.

The "carbon number of a to b" in the expression of "substituted or unsubstituted X group having a to b carbon atoms" is the carbon number of the unsubstituted X group and does not include the carbon atom(s) of an optional substituent.

The hydrogen atom referred to herein includes isotopes different from neutron numbers, i.e., light hydrogen (protium), heavy hydrogen (deuterium) and tritium.

The residues R^{1a}, R^{1b}, R^{1c}, R^{1d}, R^{2a}, R^{2b}, R^{3a}, R^{3b}, R^{3c} and R^{3d}

R^{1a}, R^{1b}, R^{1c}, R^{1d}, R^{2a}, R^{2b}, R^{3a}, R^{3b}, R^{3c} and R^{3d} are independently of each other a hydrogen atom, a substituted or unsubstituted alkyl or cycloalkyl group having 1 to 20 carbon atoms, a substituted or unsubstituted alkoxy or cycloalkoxy group having 1 to 20 carbon atoms, a substituted or unsubstituted alkenyl group having 2 to 20 carbon atoms, a substituted or unsubstituted alkynyl group having 2 to 20 carbon atoms, a substituted or unsubstituted aryl group having 6 to 30 ring carbon atoms, a substituted or unsubstituted heteroaryl group having 5 to 30 ring carbon atoms,
wherein
at least two adjacent groups, preferably two adjacent groups, R^{1a} and R^{1b}, R^{1b} and R^{1c} or R^{1c} and R^{1d}, R^{2a} and R^{2b}, and/or R^{3a} and R^{3b}, R^{3b} and R^{3c} or R^{3c} and R^{3d} are linked together to form a substituted or unsubstituted ring.

The ring formed by the at least two adjacent groups, preferably two adjacent groups, R^{1a} and R^{1b}, R^{1b} and R^{1c} or R^{1c} and R^{1d}, R^{2a} and R^{2b}, and/or R^{3a} and R^{3b}, R^{3b} and R^{3c} or R^{3c} and R^{3d} is preferably an unsaturated substituted or unsubstituted six-membered ring.

The unsaturated substituted or unsubstituted six-membered ring preferably has the following formula (A) 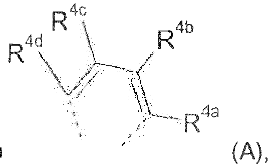 wherein
R4a, R^{4b}, R^{4c}, R^{4d} are independently of each other a hydrogen atom, a substituted or unsubstituted alkyl or cycloalkyl group having 1 to 20 carbon atoms, a substituted or unsubstituted alkoxy or cycloalkoxy group having 1 to 20 carbon atoms, a substituted or unsubstituted alkenyl group having 2 to 20 carbon atoms, a substituted or unsubstituted alkynyl group having 2 to 20 carbon atoms, a substituted or unsubstituted aryl group having 6 to 30 ring carbon atoms, a substituted or unsubstituted heteroaryl group having 3 to 30 ring carbon atoms or
two adjacent groups R^{4a} and R^{4b}, R^{4b} and R^{4c} or R^{4c} and R^{4d} may in turn be linked together to form an unsaturated six-membered ring; and
the dotted lines are boding sites.

Preferably, two adjacent groups R^{1a} and R^{1b}, R^{1b} and R^{1c}, R^{1c} and R^{1d} or R^{2a} and R^{2b} are linked together to form an unsaturated substituted or unsubstituted six-membered ring, more preferably two adjacent groups R^{1c} and R^{1d} or R^{2a} and R^{2b} are linked together to form an unsaturated substituted or unsubstituted six-membered ring. A preferred unsaturated substituted or unsubstituted six-membered ring has the formula (A) mentioned above.

Preferred residues R^{1a}, R^{1b}, R^{1c}, R^{1d}, R^{2a}, R^{2b}, R^{3a}, R^{3b}, R^{3c}, R^{3d}, R^{4a}, R^{4b}, R^{4c} and R^{4d} are independently of each other a hydrogen atom, a substituted or unsubstituted alkyl group having 1 to 20 carbon atoms, a substituted or unsubstituted aryl group having 6 to 30 ring carbon atoms, or a substituted or unsubstituted heteroaryl group having 3 to 30 ring carbon atoms, wherein
two adjacent groups, R^{1a} and R^{1b}, R^{1b} and R^{1c} or R^{1c} and R^{1d}, R^{2a} and R^{2b}, and/or R^{3a} and R^{3b}, R^{3b} and R^{3c} or R^{3c} and R^{3d}, preferably R^{1a} and R^{1b}, R^{1b} and R^{1c}, R^{1c} and R^{1d} or R^{2a} and R^{2b}, more preferably R^{1c} and R^{1d} or R^{2a} and R^{2b}, are linked together to form a substituted or unsubstituted ring. A preferred unsaturated substituted or unsubstituted six-membered ring has the formula (A) mentioned above.

More preferably, R^{1a}, R^{1b}, R^{1c}, R^{1d}, R^{2a}, R^{2b}, R^{3a}, R^{3b}, R^{3c}, R^{3d}, R^{4a}, R^{4b}, R^{4c} and R^{4d} are hydrogen,
wherein
two adjacent groups R^{1a} and R^{1b}, R^{1b} and R^{1c}, R^{1c} and R^{1d} or R^{2a} and R^{2b}, preferably R^{1c} and R^{1d} or R^{2a} and R^{2b}, are linked together to form a substituted or unsubstituted ring. A preferred unsaturated substituted or unsubstituted six-membered ring has the formula (A) mentioned above.

The polycyclic compound of formula (I) is therefore preferably represented by one of the following formulae: 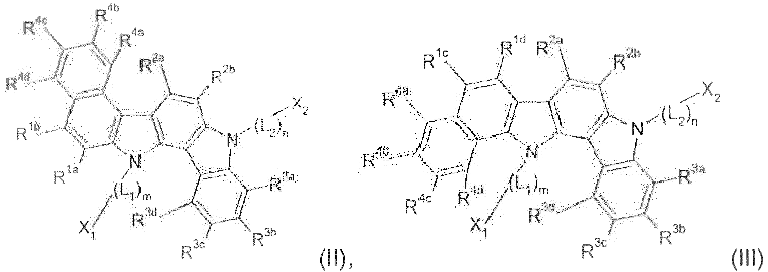 or 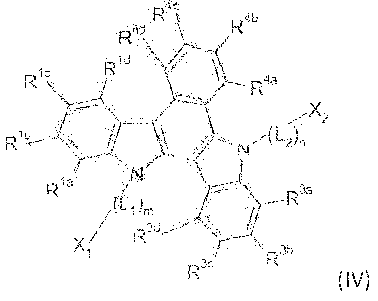 wherein the residues R^{1a}, R^{1b}, R^{1c}, R^{1d}, R^{2a}, R^{2b}, R^{3a}, R^{3b}, R^{3c}, R^{3d}, R^{4a}, R^{4b}, R^{4c}, R^{4d} and the groups L₁, L₂, X₁ and X₂ and the indices m and n are defined above and below. More preferably, the polycyclic compound of formula (I) is represented by formula (II) or (IV).

Most preferably, the polycyclic compound of formula (I) is represented by one of the following formulae: 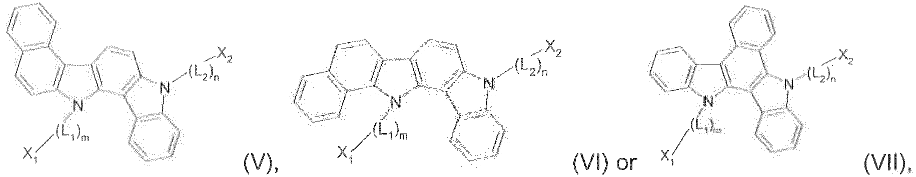 wherein the groups L₁, L₂, X₁ and X₂ and the indices m and n are defined above and below. Further most preferably, the polycyclic compound of formula (I) is represented by formula (V) or (VII).

The groups L₁, L₂, X₁ and X₂ and the indices m and n
L₁ and L₂ are each independently a direct bond, a substituted or unsubstituted arylene group having 6 to 30 ring carbon atoms, a substituted or unsubstituted heteroarylene group having 3 to 30 ring carbon atoms;
X₁ and X₂ are each independently a substituted or unsubstituted aryl group having 6 to 30 ring carbon atoms, a substituted or unsubstituted O- or S-containing heteroaryl group having 4 to 30 ring carbon atoms;
m and n are independently of each other 1 or 2,
wherein at least one of X₁ and X₂ represents a substituted or unsubstituted fluoranthene unit. Preferably, L₁ and L₂ are each independently a direct bond or a substituted or unsubstituted arylene group having 6 to 10 ring carbon atoms. More preferably, L₁ and L₂ are each independently a direct bond or a substituted or unsubstituted phenylene group, especially a 1,4-phenylene group. Most preferably, L₁ and L₂ are each independently a direct bond or an unsubstituted 1,4-phenylene group. Further most preferably, L₁ and L₂ are each a direct bond.
m and n are independently of each other 1 or 2, preferably 1. In the case that m respectively n are 1 and L is a direct bond, the group X₁ respectively the group X₂ is directly connected with the respective N atom.

X₁ and X₂ are each independently a substituted or unsubstituted aryl group having 6 to 30 ring carbon atoms, a substituted or unsubstituted O- or S-containing heteroaryl group having 4 to 30 ring carbon atoms, which is non-condensed or condensed. Preferably, X₁ and X₂ are each independently a substituted or unsubstituted aryl group having 6 to 16 ring carbon atoms, or a substituted or unsubstituted O- or S-containing heteroaryl group having 4 to 14 ring carbon atoms. More preferably, X₁ and X₂ are each independently a substituted or unsubstituted aryl group having 6 to 20, preferably 6 to 16 ring carbon atoms, wherein at least one of X₁ and X₂ represents a substituted or unsubstituted fluoranthene unit.

Suitable substituted or unsubstituted O- or S-containing heteroaryl groups having 4 to 30 ring, which are non-condensed or condensed, carbon atoms as groups X₁ and X₂ are benzofuran ring, isobenzofuran ring, benzothiophene, dibenzothiophene ring, dioxane ring, furan ring, thiophene ring, pyran ring, dibenzofuran ring and benzo[c]dibenzofuran ring, with the residues of dibenzofuran ring, dibenzothiophene ring being preferred, and the residues of dibenzofuran-2-yl group, dibenzofuran-4-yl group, dibenzothiophene-2-yl group, and dibenzothiophene-4-yl group being more preferred, wherein the rings/groups mentioned before are unsubstituted or substituted by one or more of the substituents mentioned above, preferably unsubstituted.

Suitable substituted or unsubstituted aryl groups having 6 to 30 ring carbon atoms, which are non-condensed or condensed, as groups X₁ and X₂ are phenyl group, naphthyl group, phenanthryl group, biphenyl group, terphenyl group, quaterphenyl group, fluoranthenyl group, triphenylenyl group, phenanthrenyl group, fluorenyl group, anthracenyl, chrysenyl, spirofluorenyl group, 9,9-diphenylfluorenyl group, 9,9'-spirobi[9H-fluorene]-2-yl group, 9,9-dimethylfluorenyl group, benzo[c]phenanthrenyl group, benzo[a]triphenylenyl group, naphtho[1,2-c]phenanthrenyl group, naphtho[1,2-a]triphenylenyl group, dibenzo[a,c]triphenylenyl group, and benzo[b]fluoranthenyl group, benzo[a]fluoranthenyl group, benzo[j]fluoranthenyl group, benzo[k]fluoranthenyl group, with phenyl group, naphthyl group, biphenyl group, terphenyl group, phenanthryl group, triphenylenyl group, fluorenyl group, spirobifluorenyl group, and fluoranthenyl group being preferred, and phenyl group, 1-naphthyl group, 2-naphthyl group, biphenyl-2-yl group, biphenyl-3-yl group, biphenyl-4-yl group, phenanthrene-9-yl group, phenanthrene-3-yl group, phenanthrene-2-yl group, triphenylene-2-yl group, 9,9-dimethylfluorene-2-yl group, fluoranthene-3-yl group, fluoranthene-2-yl group, fluoranthene-8-yl group being more preferred, and phenyl group, 1-naphthyl group and 2-naphthyl group being most preferred, wherein the rings/groups mentioned before are unsubstituted or substituted by one or more of the substituents mentioned above, preferably unsubstituted.

Most preferably, at least one of the groups X₁ and X₂ represents a substituted or unsubstituted fluoranthene unit and the other one - in the case that it is not a substituted or unsubstituted fluoranthene unit - represents a phenyl group, a 1-naphthyl group and a 2-naphthyl group, wherein the groups mentioned before are unsubstituted or substituted by one or more of the substituents mentioned above, preferably unsubstituted.

### Substituted or unsubstituted fluoranthene unit

At least one of, preferably one of, X₁ and X₂ in formula (I) represents a substituted or unsubstituted fluoranthene unit. Preferred fluoranthene units are 3-, 2-, 7- or 8-fluoranthene units, i.e. fluoranthene units having a bonding site in the 3-, 2-, 7- or 8- position of the fluoranthene. Most preferred are 3-, 2- and 8-fluoranthene units.

Suitable substituents in case of the substituted fluoranthene are the substituents mentioned above. Preferred substituents are a fluorine atom, a cyano group, an alkyl group having 1 to 30 carbon atoms, a silyl group, an aromatic hydrocarbon group having 6 to 30 ring carbon atoms, preferably 6 to 18 ring carbon atoms, and an heterocyclic group having 5 to 30 ring atoms, preferably 5 to 18 ring atoms; more preferably a fluorine atom, a silyl group, a cyano group, a phenyl group, a naphthyl group, a biphenyl group, a terphenyl group, a phenanthryl group, a triphenylenyl group, a fluorenyl group, a spirobifluorenyl group, a fluoranthenyl group, a residue based on a dibenzofuran ring, a residue based on a carbazole ring, a residue based on a dibenzothiophene ring, a methyl group, an ethyl group, an n-propyl group, an isopropyl group, an n-butyl group, an s-butyl group, an isobutyl group, a t-butyl group, a cyclopentyl group, and a cyclohexyl group. Most preferred substituents are a fluorine atom, a silyl group, a cyano group, a phenyl group, a naphthyl group, a biphenyl group, a methyl group, an ethyl group, an n-propyl group, an isopropyl group, an n-butyl group, an s-butyl group, an isobutyl group and a t-butyl group. Further most preferred substituents are a fluorine atom, a silyl group, a cyano group and a t-butyl group.

The number of substituents at the fluoranthene unit is generally 0,1, 2, 3 or 4, preferably 0, 1, 2 or 3, more preferably 0, 1 or 2, most preferably 0 or 1. Further most preferably, the fluoranthene unit is unsubstituted, i.e. the number of substituents is 0.

Preferably, at least one of X₁ and X₂, more preferably either X₁ or X₂, is selected from the following fluoranthene units 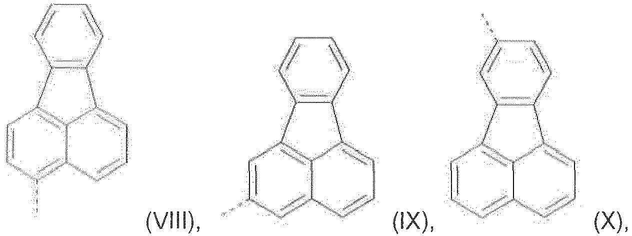 and 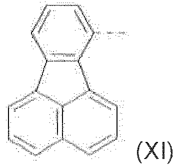 wherein the dotted line represents a bonding site. More preferably, either X₁ or X₂, is selected from the fluoranthene unit (VIII), (IX), (X).

### Compounds of formula (I)

The compounds of formula (I) as well as preferred residues, groups and indices of the compounds of formula (I) are described above.

Below, examples for compounds of formula (I) are given:

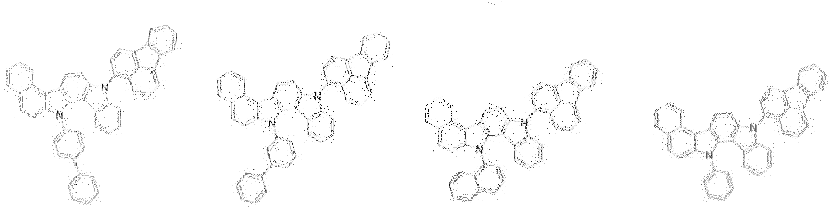

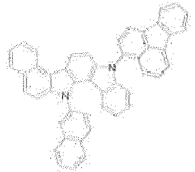

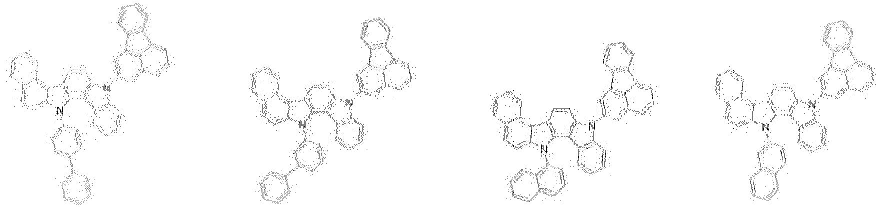

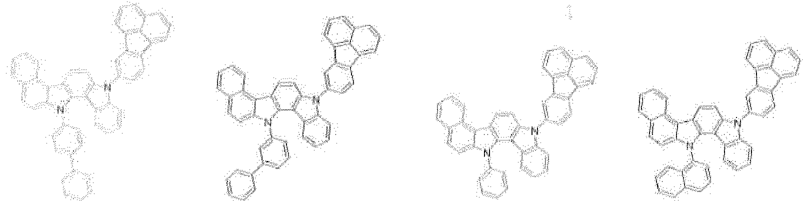

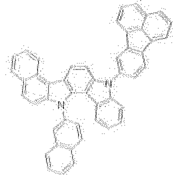

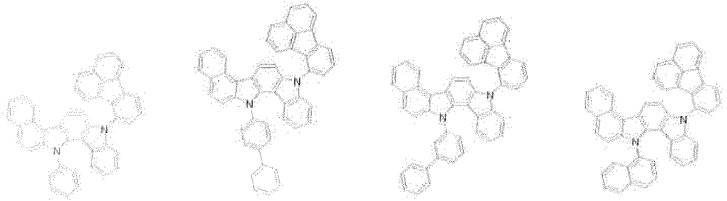

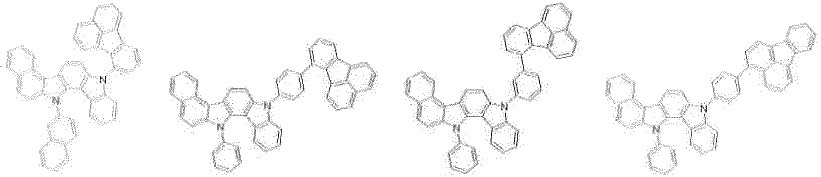

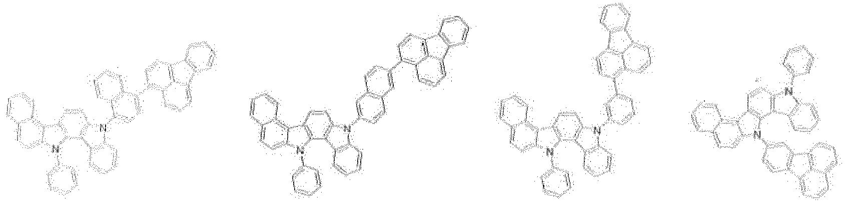

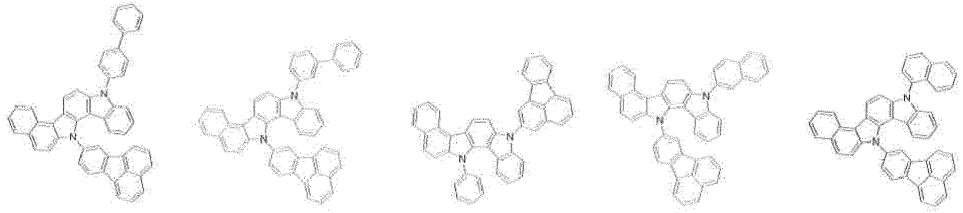

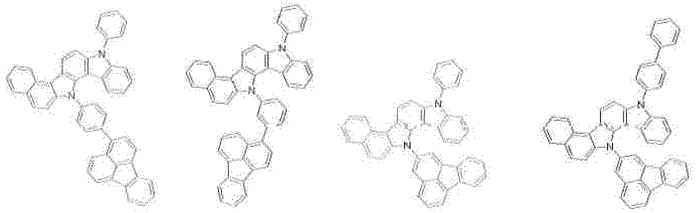

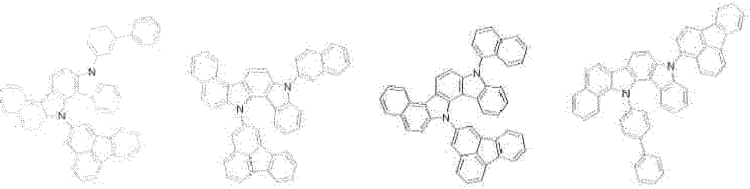

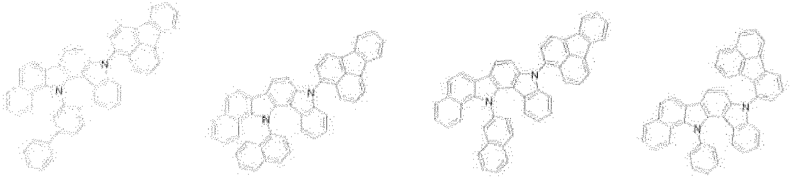

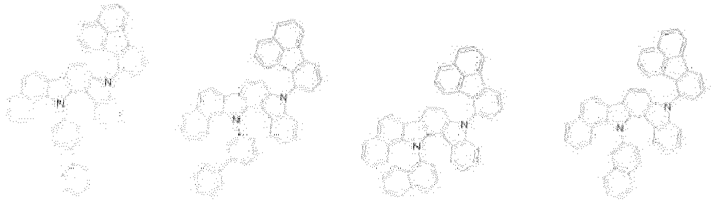

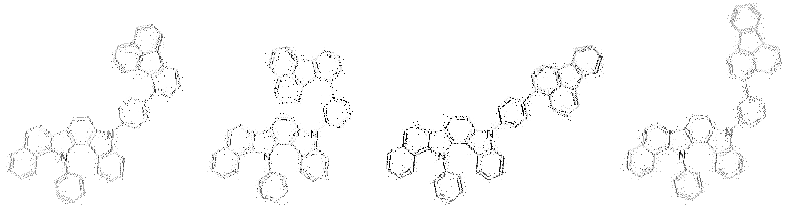

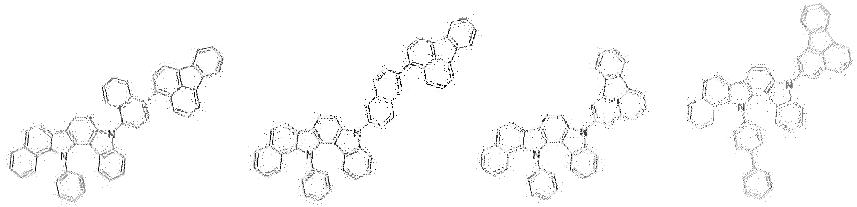

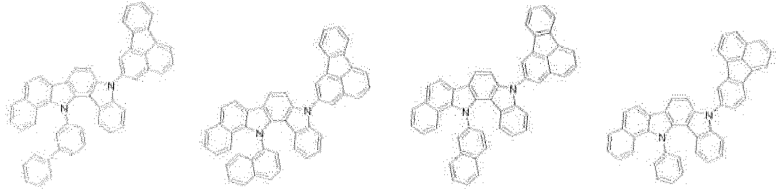

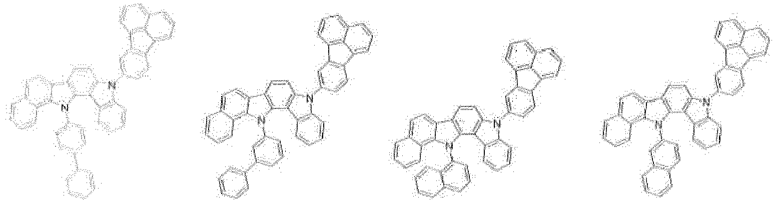

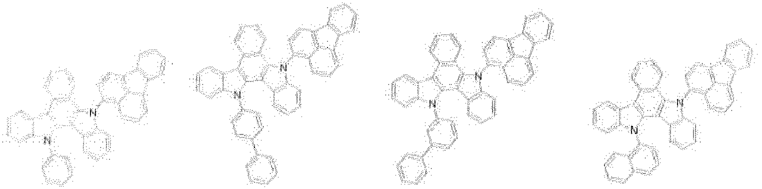

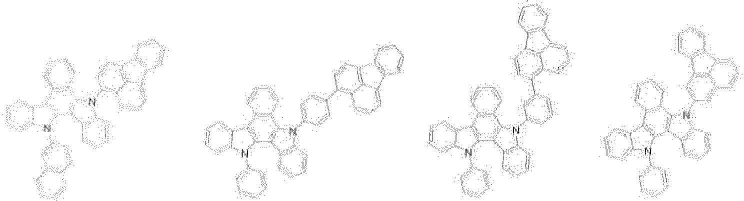

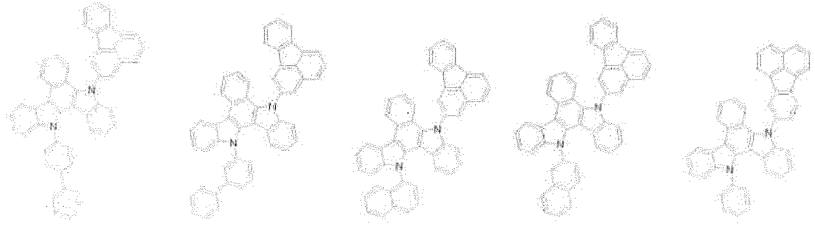

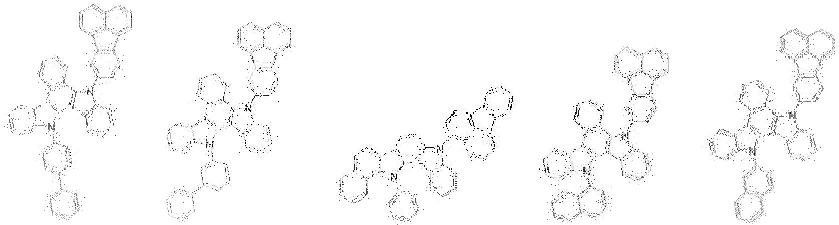

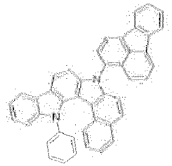

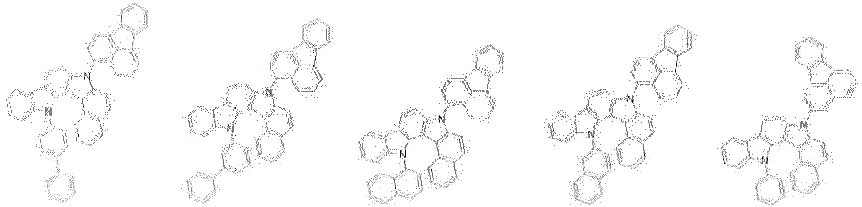

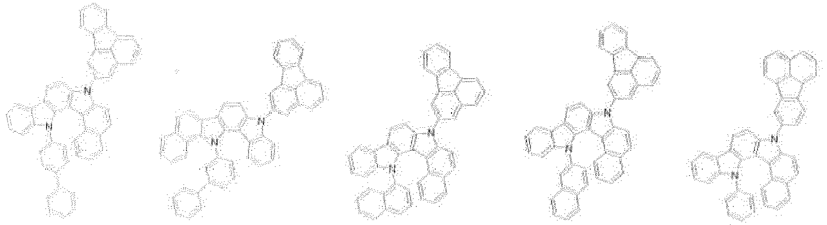

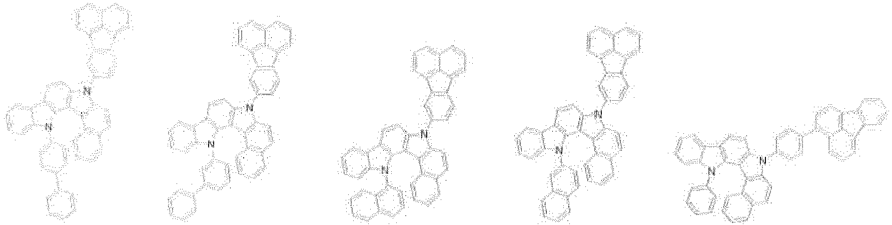

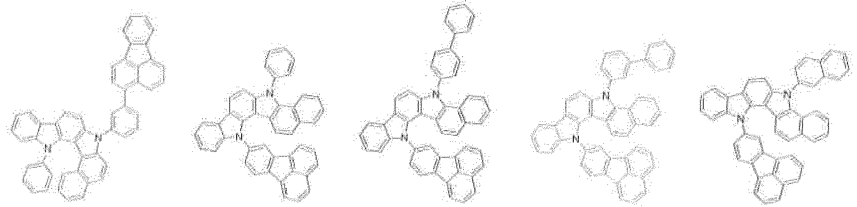

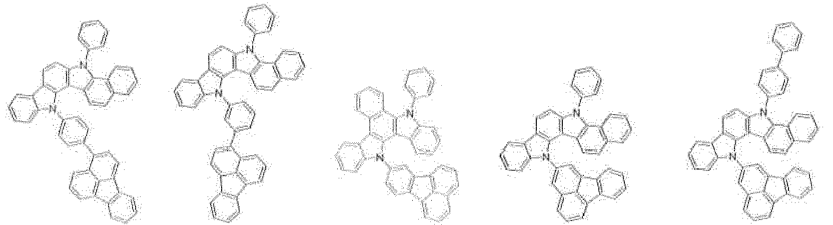

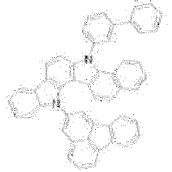

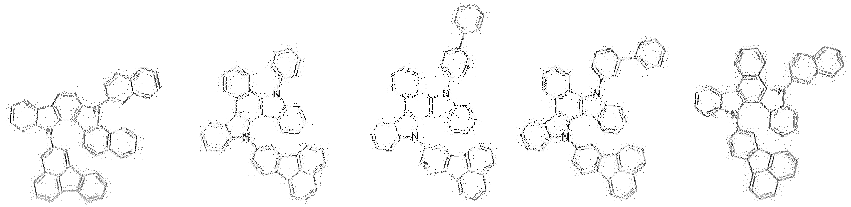

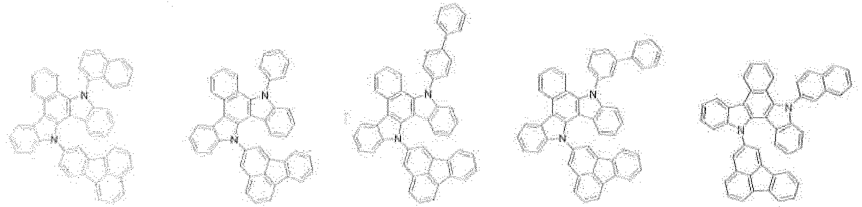

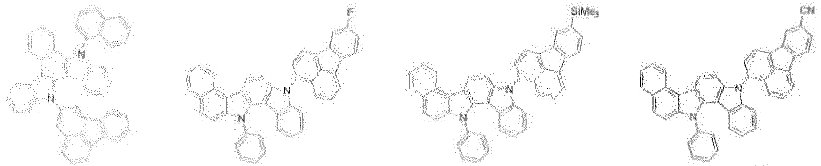

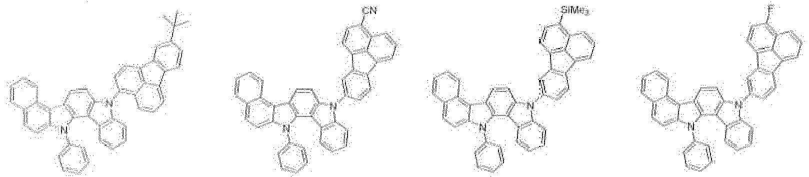

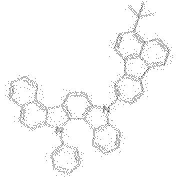

### Synthesis of the compounds of formula (I)

The compounds of formula (I) according to the present invention are for example prepared by a process at least comprising step (A):
A Coupling a compound of formula (Ia) respectively (Ib) 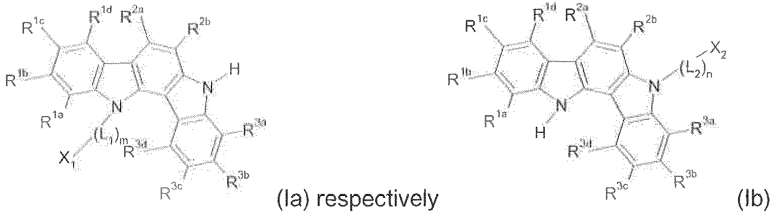 with a compound of formula 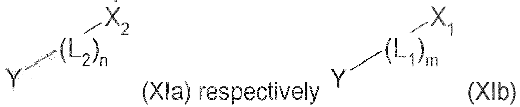 in the presence of a Pd(0) or a Pd(II) salt as a catalyst,
wherein
Y is a halide selected from the group consisting of I, F, Cl and Br, preferably Br, or a pseudohalide selected from the group consisting of mesylate, triflate, tosylate and nonaflate,
wherein the residues R^{1a}, R^{1b}, R^{1c}, R^{1d}, R^{2a}, R^{2b}, R^{3a}, R^{3b}, R^{3c}, R^{3d}, R^{4a}, R^{4b}, R^{4c}, R^{4d} and the groups L₁, L₂, X₁ and X₂ and the indices m and n are defined above.

Suitable reaction conditions for step (A) are mentioned in the examples.

The compounds of formula (Ia) and (Ib) are prepared based on processes known in the art and are for example described in WO2010/107244 A2 and KR102014098507 A.

Step (A) of the process according to the present invention can in general be conducted by any method and under any conditions that are known to provide the desired product by a person having ordinary skill in the art. For example, step (A) of the process according to the present invention can be coupling reactions that are known to a person having ordinary skill in the art, using palladium catalysts.

According to one preferred embodiment, step (A) of the process according to the present invention can be conducted using the so called Buchwald-Hartwig reaction which is known to the skilled artisan and which is, for example, mentioned in Adv. Synth. Catal., 2006, 23, J. Organomet. Chem., 1999, 125, Chem. Sci., 2011, 27.

In particular the coupling according to step (A) of the process according to the present invention is conducted in the presence of at least one basic compound, for example selected from the group consisting of alkali metal salts of alcohols having 1 to 6 carbon atoms, in particular sodium tert-butoxide, potassium tert-butoxide, potassium carbonate, cesium carbonate, rubidium carbonate or potassium phosphate.

The reaction is preferably conducted in at least one aprotic, organic solvent. Preferred are aromatic solvents, for example selected from the group consisting of toluene, benzene, xylene, mesitylene and mixtures thereof.

As a catalyst system particularly preferably a combination of at least one phosphine complex and of at least one palladium compound is used. Particularly preferably, a combination of at least one boron comprising complex and at least one palladium salt is used, for example a combination of tert-Bu₃P-HBF₄ and Pd₂(dba)₃, wherein dba means dibenzylideneacetone. Other suitable ligands and/or palladium comprising reagents are mentioned in the above mentioned scientific papers.

The reaction is conducted at a temperature that is high enough to obtain the desired compound in high yield and high selectivity, for example at 40 to 160 °C, preferably at 60 to 140 °C, particularly preferably at 100 to 130 °C.

The reaction is conducted for a time that is long enough to obtain the desired compound in high yield and high selectivity, for example for 1 to 24 h.

After the reaction is completed, the reaction mixture can be worked up according to methods that are known to the skilled artisan, for example extraction, filtration, recrystallization, chromatography etc.

The reaction product can be analyzed, for example, by proton- or carbon-NMR, mass spectrometry etc.

The substrates that are used in step (A) of the process according to the present invention, i.e. compounds according to general formula (Xla) and (Xlb) and compounds according to formula (Ia) and (Ib), can be made by methods that are known to a person having ordinary skill in the art.

Further detailed synthetic paths are given in the Examples.

### Compounds of formula (I) in organic electronics applications

It has been found that the compounds of the formula (I) are particularly suitable for use in applications in which charge carrier conductivity is required, especially for use in organic EL devices (organic electroluminescence devices).

The term organic EL device (organic electroluminescence device) is used interchangeably with the term organic light-emitting diode (OLED) in the following; i.e. both terms have the same meaning in the sense of the present application.

The present invention further relates to a material for an organic EL device comprising at least one compound of formula (I).

The compounds of formula (I) being particularly suitable in organic EL devices for use as matrix material (host material) in a light-emitting layer and/or as charge and/or exciton blocker material, i.e. as electron/exciton blocker material or as hole/exciton blocker material, and/or charge transport material, i.e. hole transport material or electron transport material, preferably as host material in a light-emitting layer and/or as electron transport material, especially in combination with a phosphorescence emitter.

In the sense of the present application, the terms matrix and host are used interchangeably,

Furthermore, the compounds of the formula (I) can be used as conductor/complementary materials in organic electronics applications selected from switching elements and organic solar cells.

### Organic EL device (OLED)

The organic EL device comprises one or more organic thin film layers including an emitting layer between a cathode and an anode, and at least one layer of the organic thin film layers comprises the compound of formula (I).

As examples of the organic thin film layers that comprise the compound of formula (I), an anode-side organic thin film layer (hole-transporting layer, hole-injecting layer, or the like), an emitting layer, a cathode-side organic thin film layer (electron-transporting layer, electron-injecting layer, or the like) provided between a cathode and an emitting layer, a spacing layer, a barrier layer or the like can be given. The examples are not limited thereto.
The compound of formula (I) may be contained in any of the abovementioned layers, and can be used as a host material or a dopant material in the emitting layer of a fluorescent emitting unit, a host material in the emitting layer of a phosphorescent emitting unit, a hole-transporting layer, an electron-transporting layer or the like of an emitting unit.

Preferably, the compounds of the formula (I) are used as matrix materials (host materials), preferably in an emitting layer of an organic EL device, more preferably in an emitting layer of an organic EL device comprising at least one compound of the formula (I) and at least one emitter material, wherein the emitter material is preferably a fluorescent or phosphorescent emitter material, more preferably a phosphorescent emitter material.

The organic EL device of the invention may be a fluorescent or phosphorescent monochromatic emitting device or may be a fluorescent/phosphorescent hybrid white emitting device. It may be a simple emitting device having a single emitting unit or a tandem emitting device having plural emitting units. Among them, the organic EL device may preferably be a phosphorescent emitting device.

As the representative device structure of a simple type organic EL device, the following device configuration can be given.

### (1) Anode/emitting unit/cathode

The emitting unit mentioned above may be a stacked type emitting unit comprising plural phosphorescent emitting layers or plural fluorescent emitting layers. In this case, in order to prevent diffusion of excitons generated in the phosphorescent emitting layer to the fluorescent emitting layer, a spacing layer may be provided between the emitting layers. The representative layer configuration of the emitting unit is given below.
(a) Hole-transporting layer/Emitting layer (/Electron-transporting layer)
(b) Hole-transporting layer/First phosphorescent emitting layer/Second phosphorescent emitting layer (/Electron-transporting layer)
(c) Hole-transporting layer/Phosphorescent emitting layer/Spacing layer/Fluorescent emitting layer (/Electron-transporting layer)
(d) Hole-transporting layer/First phosphorescent emitting layer/Second phosphorescent emitting layer/Spacing layer/Fluorescent emitting layer (/Electron-transporting layer)
(e) Hole-transporting layer/First phosphorescent emitting layer/Spacing layer/Second phosphorescent emitting layer/Spacing layer/Fluorescent emitting layer (/Electron-transporting layer)
(f) Hole-transporting layer/Phosphorescent emitting layer/Spacing layer/First fluorescent emitting layer/Second fluorescent emitting layer (/Electron-transporting layer)
(g) Hole-transporting layer/Electron barrier layer/Emitting layer (/Electron-transporting layer)
(h) Hole-transporting layer/Emitting layer/Hole barrier layer (/Electron-transporting layer)
(i) Hole-transporting layer/Fluorescent emitting layer/Triplet barrier layer (/Electron-transporting layer)
The phosphorescent or fluorescent emitting layer as mentioned above can emit different colors of light. Specifically, in the stacked emitting layer (d), a layer configuration of the hole transporting layer/first phosphorescent emitting layer (red emission)/second phosphorescent emitting layer (green emission)/spacing layer/fluorescent emitting layer (blue emission)/electron transporting layer or the like can be given.

Between each emitting layer and the hole-transporting layer or the spacing layer, an electron barrier layer may be provided appropriately. Between each emitting layer and the electron transporting layer, a hole-barrier layer (a hole blocking layer) may be provided appropriately. Due to provision of an electron-barrier layer or a hole-barrier layer, electrons or holes can be confined within the emitting layer, whereby possibility of recombination of carriers in the emitting layer can be increased, and the life can be improved.

As the represented device configuration of a tandem organic EL device, the following device configuration can be given.

### (2) Anode/first emitting unit/intermediate layer/second emitting unit/cathode

Here, as the first emitting unit and the second emitting unit, the same emitting units as those mentioned above can independently be given, for example.
In general, the intermediate layer is called an intermediate electrode, an intermediate conductive layer, a carrier-generating layer, an electron-withdrawing layer, and a known material configuration that supplies electrons to the first emitting unit and supplies holes to the second emitting unit can be used.

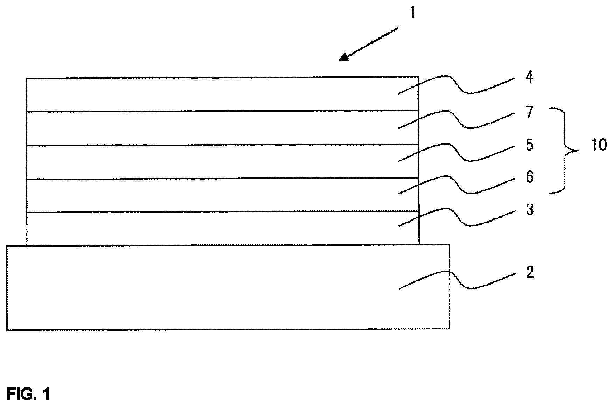

A schematic structure of an example of the OLED in an aspect of the invention is for example shown in FIG. 1 wherein the OLED 1 comprises a substrate 2, an anode 3, a cathode 4, and an emission unit 10 disposed between the anode 3 and the cathode 4. The emission unit 10 comprises a light emitting layer 5 which comprises at least one emitting layer, preferably at least one phosphorescent emitting layer, containing a host material, preferably a phosphorescent host material, and a dopant material, preferably a phosphorescent dopant material (phosphorescent material). A hole injecting/transporting layer (an anode-side organic thin film layer) 6 may be disposed between the light emitting layer 5 and the anode 3, and an electron injecting/transporting layer (a cathode-side thin film layer) 7 may be disposed between the light emitting layer 5 and the cathode 4. An electron blocking layer may be disposed on the anode 3 side of the light emitting layer 5, and a hole blocking layer may be disposed on the cathode 4 side of the light emitting layer 5. With these blocking layers, electrons and holes are confined in the light emitting layer 5 to increase the degree of exciton generation in the light emitting layer 5.

Herein, a host that is combined with a fluorescent dopant is referred to as a fluorescent host and a host that is combined with a phosphorescent dopant is referred to as a phosphorescent host. The fluorescent host and the phosphorescent host are not distinguished only by the molecular structure thereof. That is, the phosphorescent host means a material constituting a phosphorescent emitting layer that contains a phosphorescent dopant and does not mean a material that cannot be used as a material constituting a fluorescent dopant. The same can be applied to a fluorescent host.

### Substrate

The organic EL device is usually formed on a transparent substrate. The transparent substrate is a substrate for supporting the organic EL device, and is preferably a flat and smooth substrate having a 400-to-700-nm-visible-light transmittance of 50% or more. Specific examples thereof include glass plates and polymer plates. Examples of the glass plate include those obtained by using as raw materials soda-lime glass, barium/strontium-containing glass, lead glass, aluminosilicate glass, borosilicate glass, barium borosilicate glass, quartz, or the like. Examples of the polymer plate include those obtained by using as raw materials polycarbonate, acrylic polymer, polyethylene terephthalate, polyethersulfide, polysulfone, or the like.

### Anode

The anode is an electrode which provides positive charge carriers. It may be composed, for example, of materials which comprise a metal, a mixture of different metals, a metal alloy, a metal oxide or a mixture of different metal oxides. Alternatively, the anode may be a conductive polymer. Suitable metals comprise the metals of groups 11, 4, 5 and 6 of the Periodic Table of the Elements, and also the transition metals of groups 8 to 10. When the anode is to be transparent, mixed metal oxides of groups 12, 13 and 14 of the Periodic Table of the Elements are generally used, for example indium tin oxide (ITO). It is likewise possible that the anode (a) comprises an organic material, for example polyaniline, as described, for example, in Nature, Vol. 357, pages 477 to 479 (June 11, 1992). Preferred anode materials include conductive metal oxides, such as indium tin oxide (ITO) and indium zinc oxide (IZO), aluminum zinc oxide (AlZnO), and metals. Anode (and substrate) may be sufficiently transparent to create a bottom-emitting device. A preferred transparent substrate and anode combination is commercially available ITO (anode) deposited on glass or plastic (substrate). A reflective anode may be preferred for some top-emitting devices, to increase the amount of light emitted from the top of the device. At least either the anode or the cathode should be at least partly transparent in order to be able to emit the light formed. Other anode materials and structures may be used.

### Hole-transporting layer

The hole-transporting layer is an organic layer that is formed between the emitting layer and the anode, and has a function of transporting holes from the anode to the emitting layer. If the hole-transporting layer is composed of plural layers, an organic layer that is nearer to the anode may often be defined as the hole-injecting layer. The hole-injecting layer has a function of injecting holes efficiently to the organic layer unit from the anode.

Said hole injection layer is generally used for stabilizing hole injection from anode to hole transporting layer which is generally consist of organic materials.
Organic material having good contact with anode or organic material with p-type doping is preferably used for the hole injection layer.
Acceptor materials, or fused aromatic hydrocarbon materials or fused heterocycles which have high planarity, are preferably used, acceptor materials are more preferably used for the hole injection layer.
Specific examples for acceptor materials are, the quinone derivatives with one or more electron withdrawing groups, such as F₄TCNQ(2,3,5,6-tetrafluoro-7,7,8,8-tetracyanoquinodimethane), 1,2,3-Tris[(cyano)(4-cyano-2,3,5,6-tetrafluorophenyl)methylene]cyclopropane, and so on; hexa-azatriphenylene derivatives with one or more electron withdrawing groups, such as hexa-azatriphenylene-hexanitrile; aromatic hydrocarbon derivatives with one or more electron withdrawing groups; aryl boron derivatives with one or more electron withdrawing groups, and so on.
p-doping is usually consist of one or more p-dopant materials and one or more matrix materials. Matrix materials preferably have shallower HOMO level and p-dopant preferably have deeper LUMO level to enhance the carrier density of the layer. Aryl or heteroaryl amine derivatives are preferably used as the matrix materials. Specific examples for the matrix material are the same as that for hole transporting layer which is explained at the later part. Specific examples for p-dopant are the above mentioned acceptor materials, preferably the quinone derivatives with one or more electron withdrawing groups, such as F₄TCNQ, 1,2,3-Tris[(cyano)(4-cyano-2,3,5,6-tetrafluorophenyl)methylene]cyclopropane.
The ratio of the p-type dopant is preferably less than 20% of molar ratio, more preferably less than 10%, such as 1%, 3%, 5% and so on.

Hole transporting layer is generally used for injecting and transporting holes efficiently, and aromatic or heterocyclic amine derivatives are preferably used.
Specific examples for hole transporting layer are represented as general formula (H),

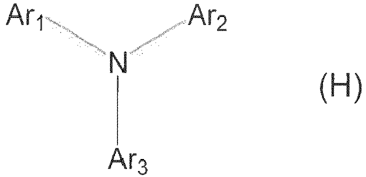

Ar₁∼Ar₃ each independently represents substituted or unsubstituted aryl group having 5 to 50 carbon atoms or substituted or unsubstituted heterocyclic group having 5 to 50 cyclic atoms, preferably phenyl group, biphenyl group, terphenyl group, naphthyl group, phenanthryl group, triphenylenyl group, fluorenyl group, spirobifluorenyl group, indenofluorenyl group, carbazolyl group, dibenzofuranyl group, dibenzothiophenyl group, carbazole substituted aryl group, dibenzofuran substituted aryl group or dibenzothiophene substituted aryl group; two or more substituents selected among Ar¹∼Ar³ may be bonded to each other to form a ring structure, such as carbazole ring structure, acridane ring structure and so on.
According to one embodiment, it is preferable that at least one of Ar₁∼Ar₃ have additional one aryl or heterocyclic amine substituent, more preferably Ar1 have an additional aryl amino substituent, at the case of that it is preferable that Ar1 represents substituted or unsubstituted biphenylene group, substituted or unsubstituted fluorenylene group.

Second hole transporting layer is usually inserted between the first hole transporting layer and the emitting layer to enhance device performance by blocking excess electrons or excitons. Specific examples for second hole transporting layer is the same as the first hole transporting layer. It is preferably that second hole transporting layer have higher triplet energy to block triplet exciton especially for phosphorescent green device, such as bicarbazole derivatives, biphenylamine derivatives, triphenylenyl amine derivatives, fluorenyl amine detrivatives, carbazole substituted arylamine derivatives, dibenzofuran substituted arylamine derivatives, dibenzothiophene substituted arylamine derivatives, and so on.

### Exciton blocking layer

Blocking layers may be used to reduce the number of charge carriers (electrons or holes) and/or excitons that leave the emissive layer. An electron/exciton blocking layer (d) may be disposed between the first emitting layer (e) and the hole transport layer (c), to block electrons from emitting layer (e) in the direction of hole transport layer (c). Blocking layers may also be used to block excitons from diffusing out of the emissive layer.

Suitable metal complexes for use as electron/exciton blocker material are, for example, carbene complexes as described in WO2005/019373A2, WO2006/056418A2, WO2005/113704, WO2007/115970, WO2007/115981, WO2008/000727 and PCT/EP2014/055520. Explicit reference is made here to the disclosure of the WO applications cited, and these disclosures shall be considered to be incorporated into the content of the present application.

According to a preferred embodiment of the present invention, at least one compound of general formula (I) is present in the exciton blocking layer of the OLED according to the present invention.

### Emitting layer

The light emitting layer is an organic layer having a light emitting function and is formed from one or more layers, wherein one of the layers comprises a host material (first host material), optionally a second host material, and the light emitting material.

According to a preferred embodiment of the present invention, at least one compound of general formula (I) is present in the emitting layer of the OLED according to the present invention, preferably as host material.

When the light emitting layer is composed of two or more layers, the light emitting layer or layers other than that mentioned above contains or contain a host material and a dopant material when a doping system is employed. The major function of the host material is to promote the recombination of electrons and holes and confine excitons in the light emitting layer. The dopant material causes the excitons generated by recombination to emit light efficiently.

In case of a phosphorescent device, the major function of the host material is to confine the excitons generated on the dopant in the light emitting layer.

The light emitting layer may be made into a double dopant layer, in which two or more kinds of dopant materials having high quantum yield are used in combination and each dopant material emits light with its own color. For example, to obtain a yellow emission, a light emitting layer formed by co-depositing a host, a red-emitting dopant and a green-emitting dopant is used.

In a laminate of two or more light emitting layers, electrons and holes are accumulated in the interface between the light emitting layers, and therefore, the recombination region is localized in the interface between the light emitting layers, to improve the quantum efficiency.

The light emitting layer may be different in the hole injection ability and the electron injection ability, and also in the hole transporting ability and the electron transporting ability each being expressed by mobility.

The light emitting layer is formed, for example, by a known method, such as a vapor deposition method, a spin coating method, and LB method. Alternatively, the light emitting layer may be formed by making a solution of a binder, such as resin, and the material for the light emitting layer in a solvent into a thin film by a method such as spin coating.

The light emitting layer is preferably a molecular deposit film. The molecular deposit film is a thin film formed by depositing a vaporized material or a film formed by solidifying a material in the state of solution or liquid. The molecular deposit film can be distinguished from a thin film formed by LB method (molecular build-up film) by the differences in the assembly structures and higher order structures and the functional difference due to the structural differences.

The light-emitting layer (e) comprises at least one fluorescence or phosphorescence emitter, suitable emitter materials being known to those skilled in the art.

The emission wavelength of the phosphorescent dopant used in the light emitting layer is not particularly limited. In a preferred embodiment, at least one of the phosphorescent dopants used in the light emitting layer has the peak of emission wavelength of in general 430 nm or longer and 780 nm or shorter, preferably 490 nm or longer and 700 nm or shorter and more preferably 490 nm or longer and 650 nm or shorter. Most preferred are green emitter materials (490 to 570 nm). In another preferred embodiment, red emitter materials (570 to 680 nm) are preferred.

The phosphorescent dopant (phosphorescent emitter material) is a compound which usually emits light by releasing the energy of excited triplet state.
The compounds according to general formula (I) are most preferably used as the matrix (=host material) in the light-emitting layer.

Suitable metal complexes for use in the inventive OLEDs, preferably as emitter material, are described, for example, in documents WO 02/60910 A1, US 2001/0015432 A1, US 2001/0019782 A1, US 2002/0055014 A1, US 2002/0024293 A1, US 2002/0048689 A1, EP 1 191 612 A2, EP 1 191 613 A2, EP 1 211 257 A2, US 2002/0094453 A1, WO 02/02714 A2, WO 00/70655 A2, WO 01/41512 A1, WO 02/15645 A1, WO 2005/019373 A2, WO 2005/113704 A2, WO 2006/115301 A1, WO 2006/067074 A1, WO 2006/056418, WO 2006121811 A1, WO 2007095118 A2, WO 2007/115970, WO 2007/115981, WO 2008/000727, WO2010129323, WO2010056669, WO10086089, US2011/0057559, WO2011/106344, US2011/0233528, WO2012/048266 and WO2012/172482.

Further suitable metal complexes are the commercially available metal complexes tris(2-phenylpyridine)iridium(III), iridium(III) tris(2-(4-tolyl)pyridinato-N,C^{2'}), bis(2-phenylpyridine)(acetylacetonato)iridium(III), iridium(III) tris(1-phenylisoquinoline), iridium(III) bis(2,2'-benzothienyl)pyridinato-N,C^{3'})(acetylacetonate), tris(2-phenylquinoline)iridium(III), iridium(III) bis(2-(4,6-difluorophenyl)pyridinato-N,C²)picolinate, iridium(III) bis(1-phenylisoquinoline)(acetylacetonate), bis(2-phenylquinoline)(acetylacetonato)iridium(III), iridium(III) bis(di-benzo[f,h]quinoxaline)(acetylacetonate), iridium(III) bis(2-methyldi-benzo[f,h]quinoxaline)(acetylacetonate) and tris(3-methyl-1-phenyl-4-trimethylacetyl-5-pyrazolino)terbium(III), bis[1-(9,9-dimethyl-9H-fluoren-2-yl)isoquinoline](acetylacetonato)iridium(III), bis(2-phenylbenzothiazolato)(acetylacetonato)iridium(III), bis(2-(9,9-dihexylfluorenyl)-1-pyridine)(acetylacetonato)iridium(III), bis(2-benzo[b]thiophen-2-yl-pyridine) (acetylacetonato)iridium(III).

In addition, the following commercially available materials are suitable:
tris(dibenzoylacetonato)mono(phenanthroline)europium(III), tris(dibenzoylmethane)-mono(phenanthroline)europium(III), tris(dibenzoylmethane)mono(5-aminophenanthroline)-europium(III), tris(di-2-naphthoylmethane)mono(phenanthroline)europium(III), tris(4-bromobenzoylmethane)mono(phenanthroline)europium(III), tris(di(biphenyl)methane)mono(phenanthroline)europium(III), tris(dibenzoylmethane)mono(4,7-diphenylphenanthroline)europium(III), tris(dibenzoylmethane)mono(4,7-di-methylphenanthroline)europium(III), tris(dibenzoylmethane)mono(4,7-dimethylphenanthrolinedisulfonic acid)europium(III) disodium salt, tris[di(4-(2-(2-ethoxyethoxy)ethoxy)benzoylmethane)]mono(phenanthroline)europium(III) and tris[di[4-(2-(2-ethoxyethoxy)ethoxy)benzoylmethane)]mono(5-aminophenanthroline)europium(III), osmium(II) bis(3-(trifluoromethyl)-5-(4-tert-butylpyridyl)-1,2,4-triazolato)diphenylmethylphosphine, osmium(II) bis(3-(trifluoromethyl)-5-(2-pyridyl)-1,2,4-triazole)dimethylphenylphosphine, osmium(II) bis(3-(trifluoromethyl)-5-(4-tert-butylpyridyl)-1,2,4-triazolato)dimethylphenylphosphine, osmium(II) bis(3-(trifluoromethyl)-5-(2-pyridyl)pyrazolato)dimethylphenylphosphine, tris[4,4'-di-tert-butyl(2,2')-bipyridine]ruthenium(III), osmium(II) bis(2-(9,9-dibutylfluorenyl)-1-isoquinoline(acetylacetonate).

Particularly suitable metal complexes are described in US2014048784, US2012223295, US2014367667, US2013234119, US2014001446, US2014231794, US2014008633, WO2012108388 and WO2012108389. The emitters mentioned in US2013234119, paragraph [0222], are exemplified. Selected emitters, especially red emitters, of said emitters mentioned in US2013234119, paragraph [0222], are:

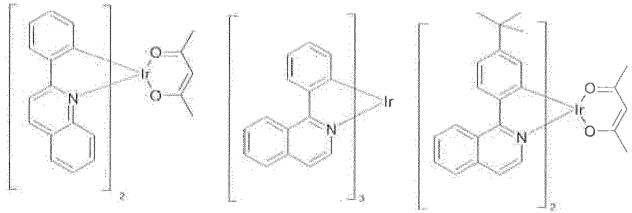

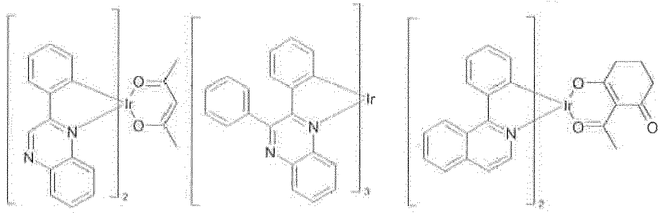

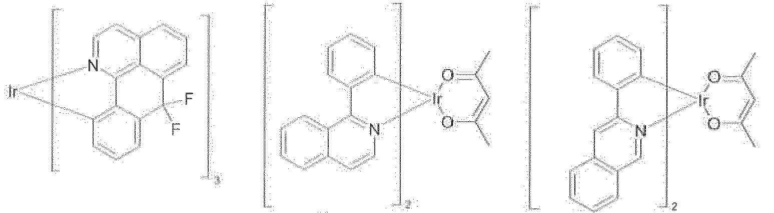

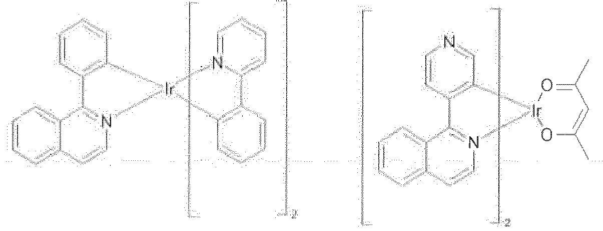

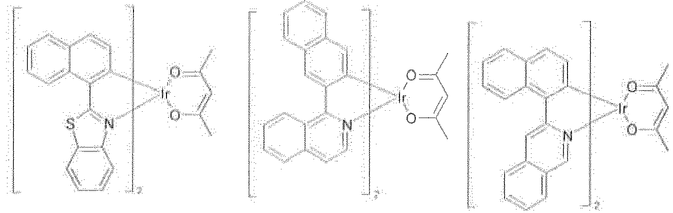

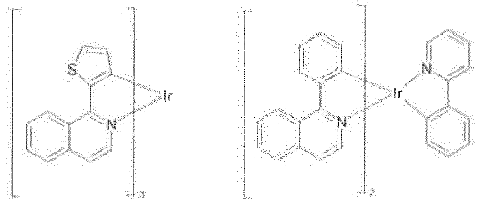

Further suitable Emitters are mentioned in: Mrs Bulletin, 2007, 32, 694:

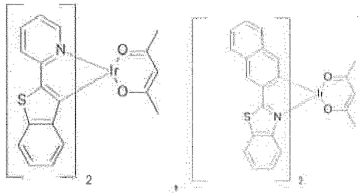

Further suitable Emitters are mentioned in: WO2009100991:

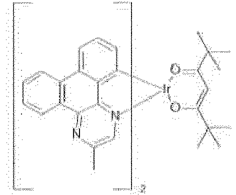

Further suitable Emitters are mentioned in: WO2008101842:

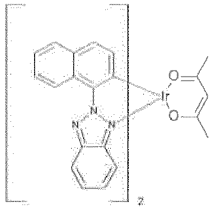

Further suitable Emitters are mentioned in: US 20140048784, especially in paragraph [0159]:

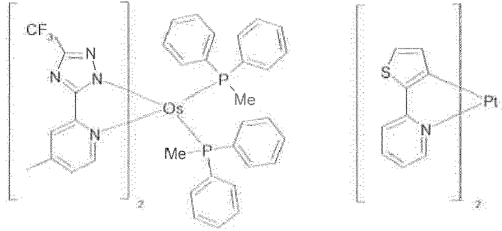

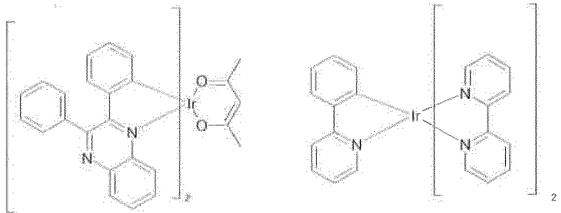

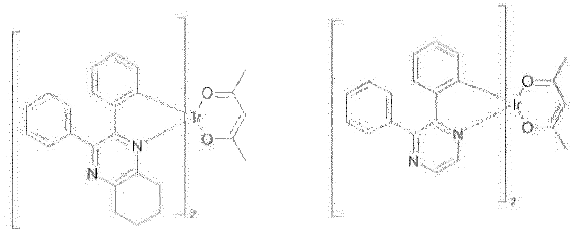

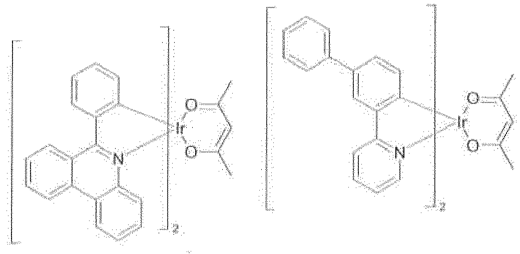

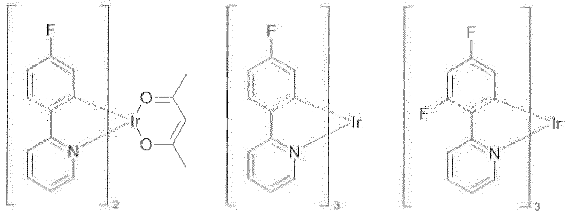

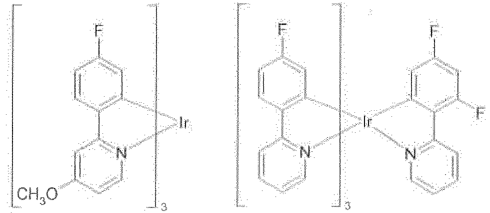

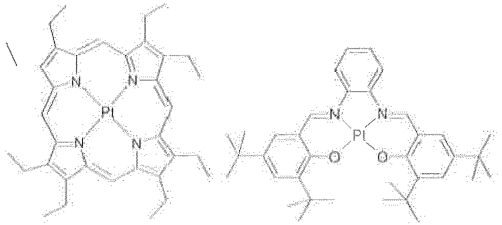

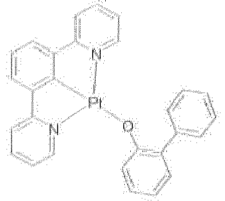

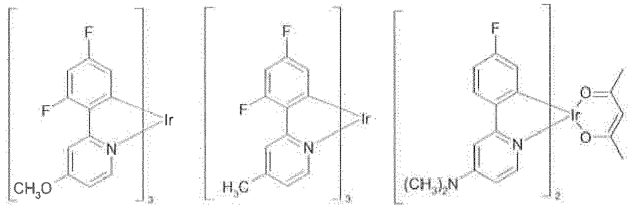

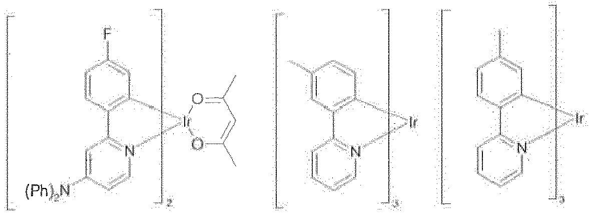

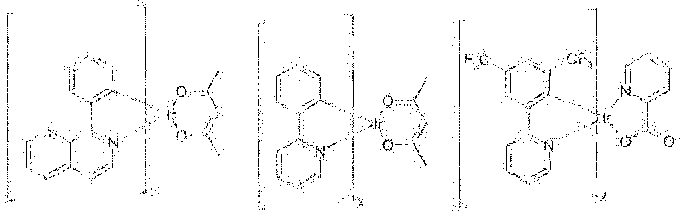

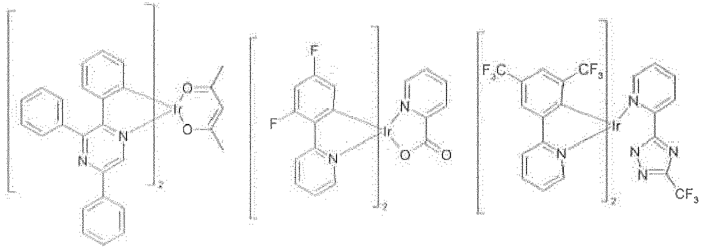

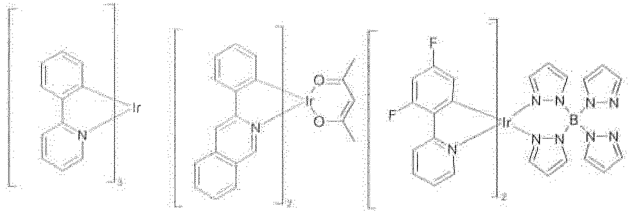

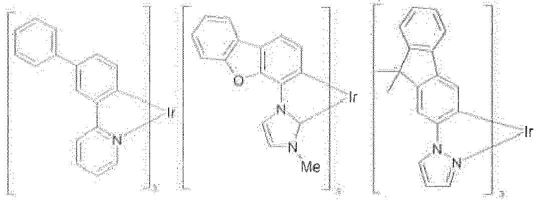

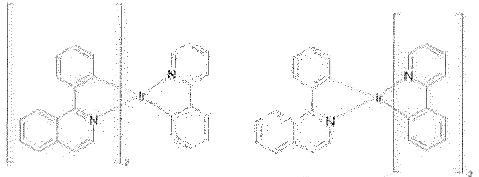

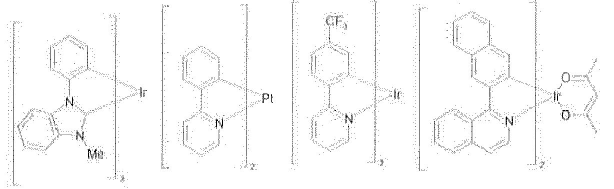

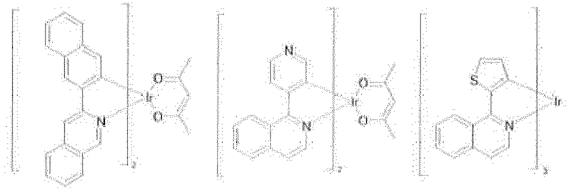

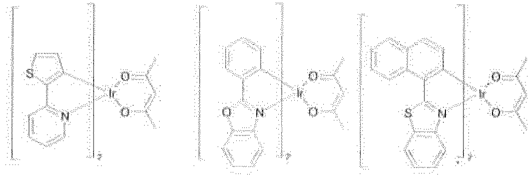

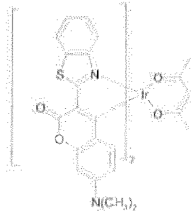

Further suitable red emitters are shown in WO 2008/109824. Preferred red emitters according to this document are the following compounds:

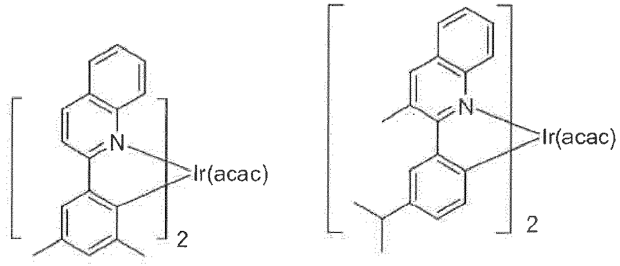

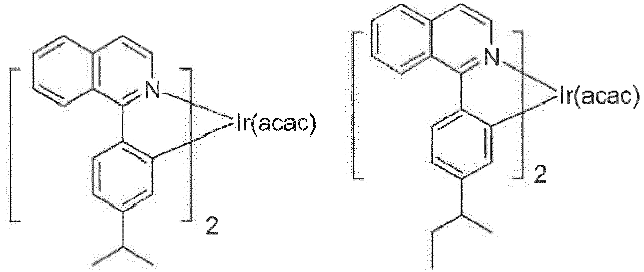

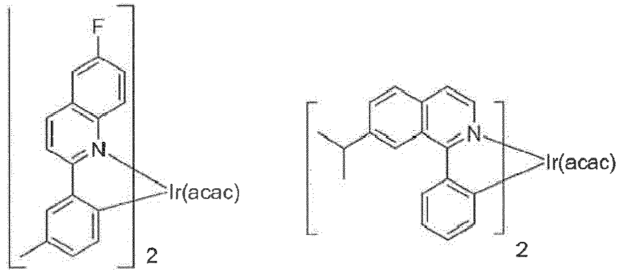

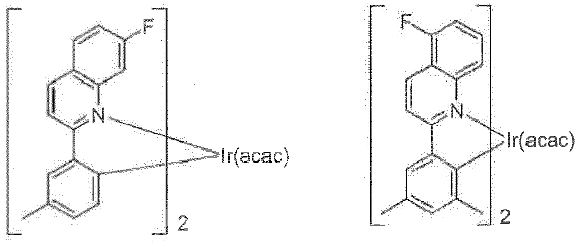

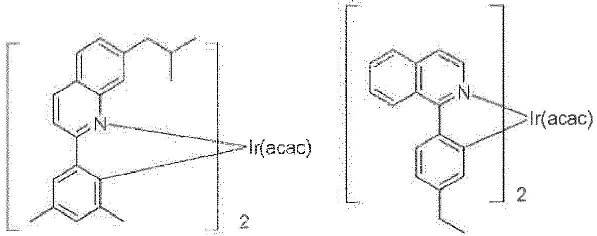

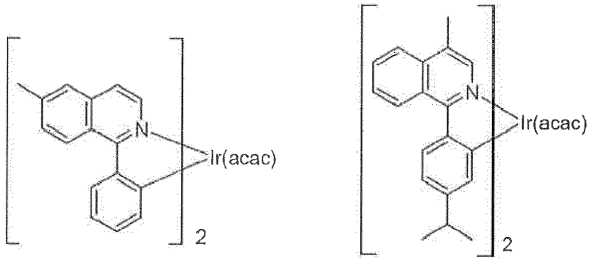

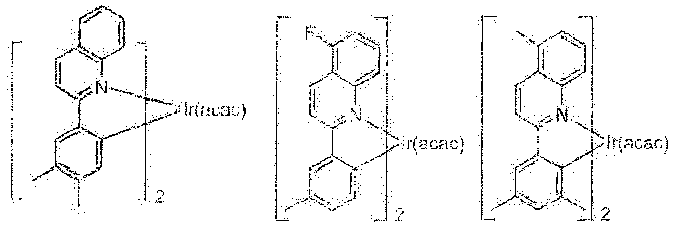

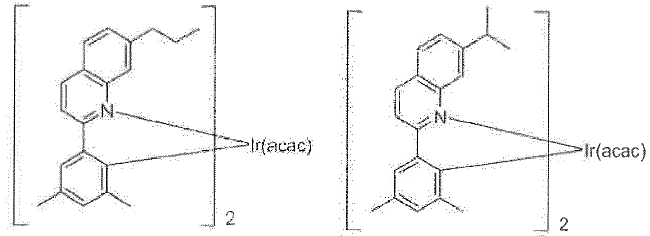

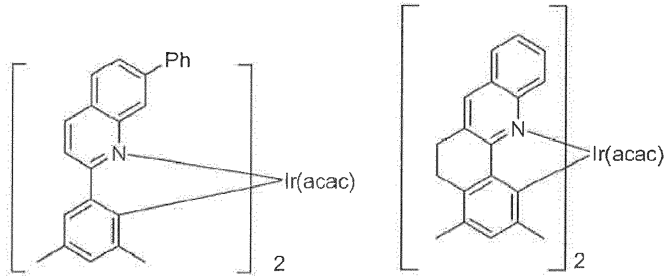

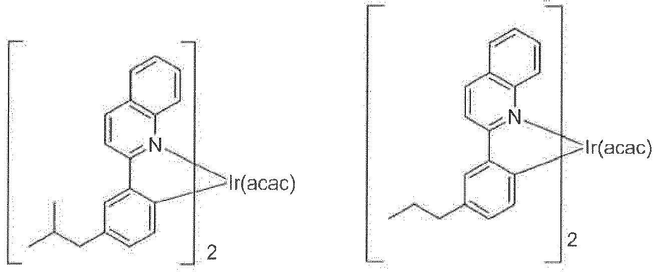

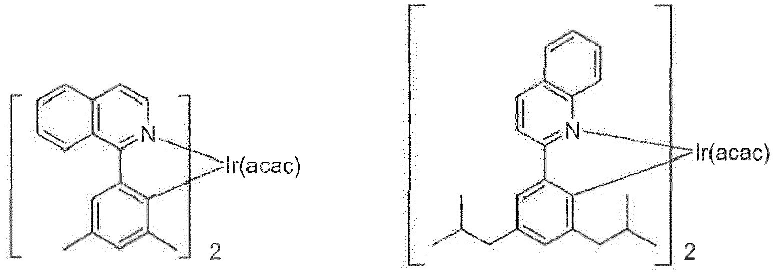

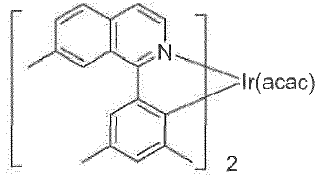

The emitter materials (dopants), preferably the phosphorescent emitter materials, may be used alone or in combination of two or more.

Further red emitters that may be used in the OLEDs according to the present invention are disclosed in US 2013/0299795 and are shown in the following:

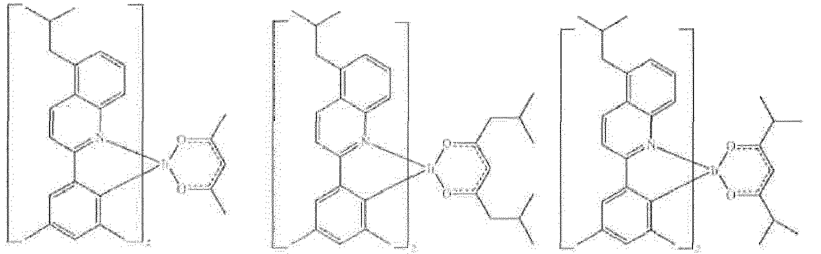

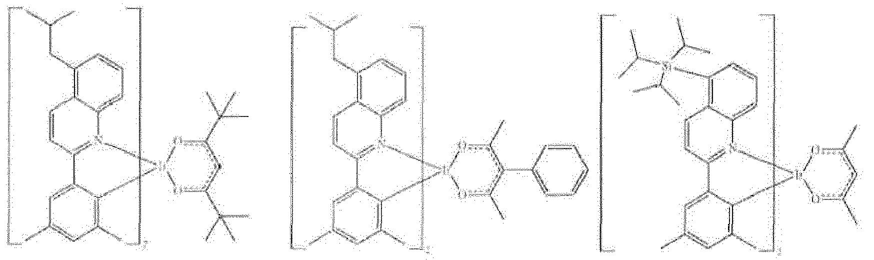

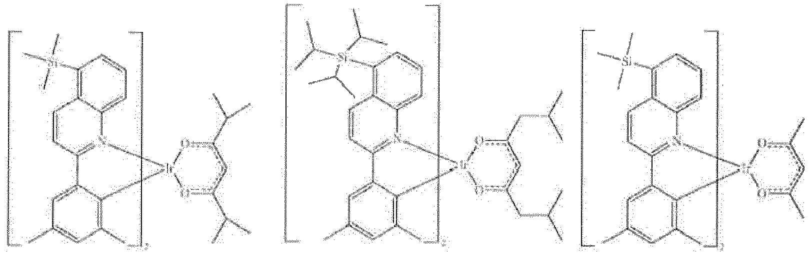

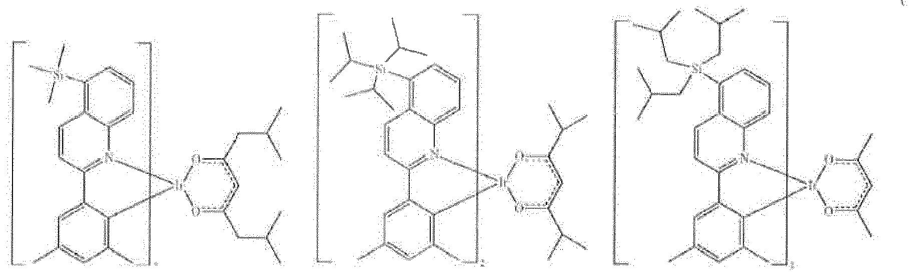

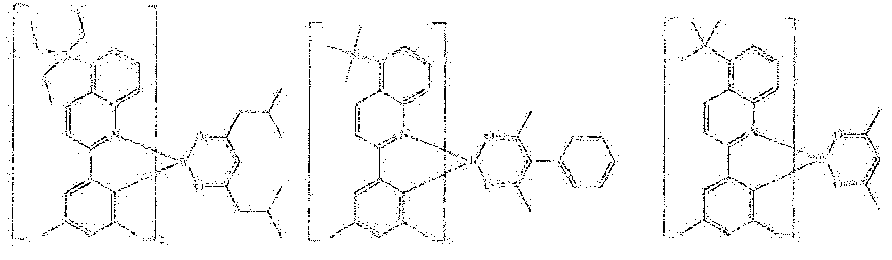

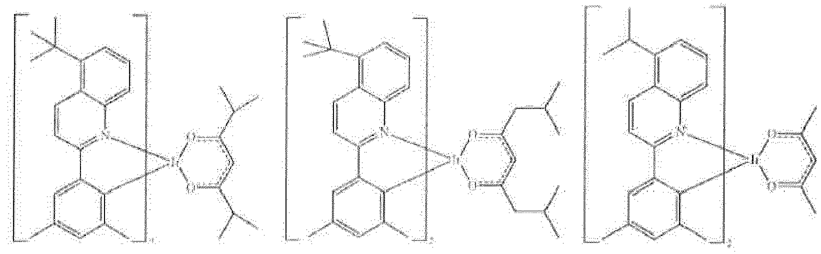

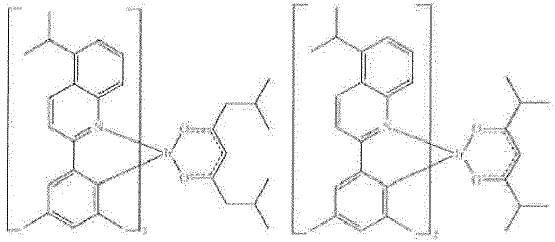

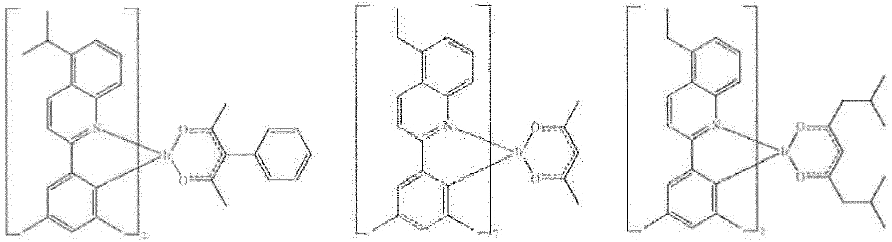

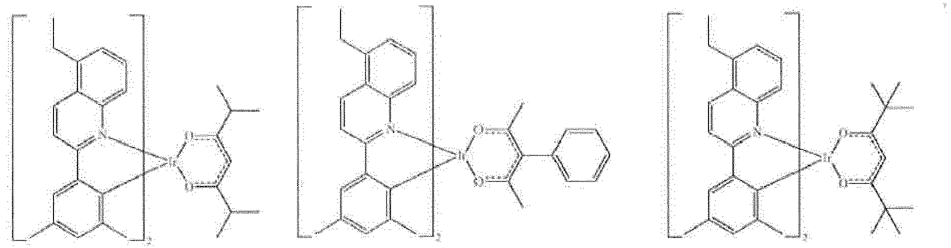

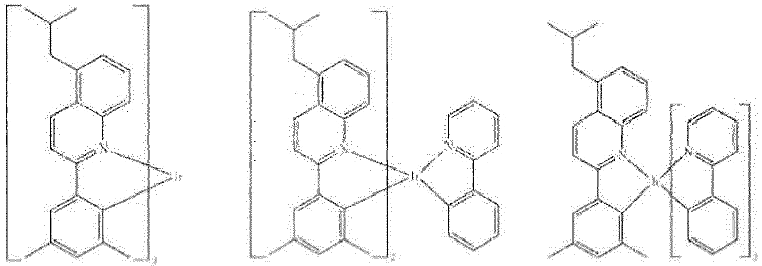

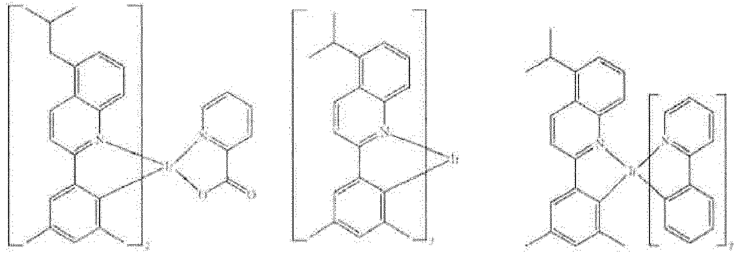

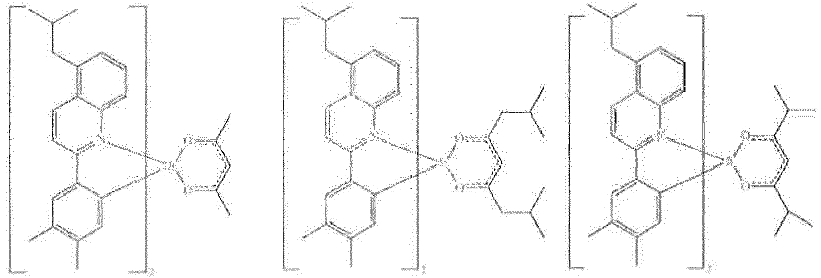

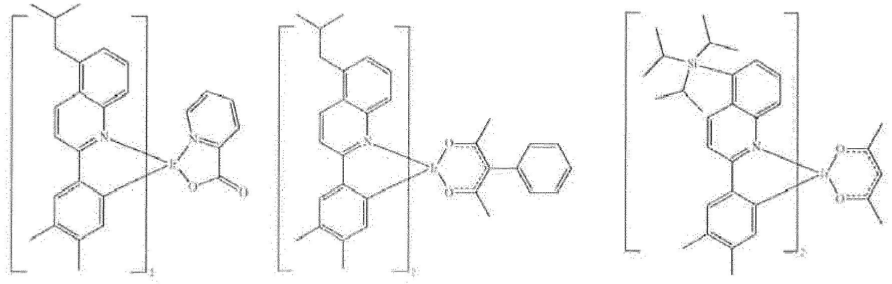

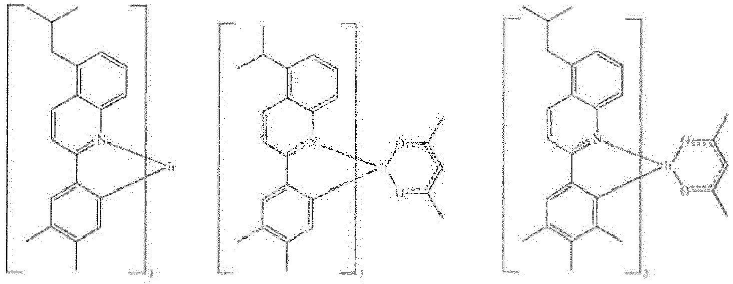

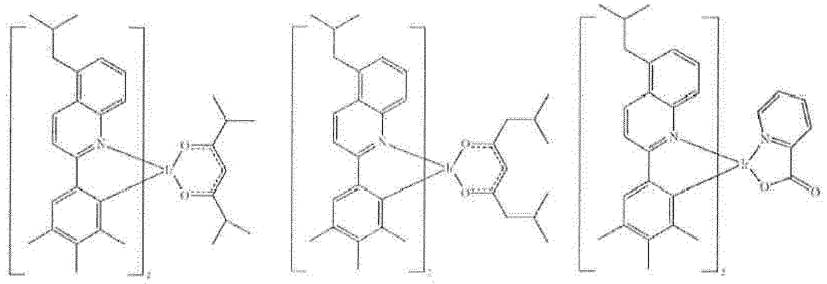

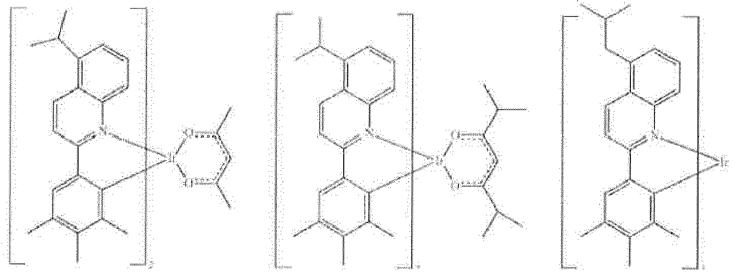

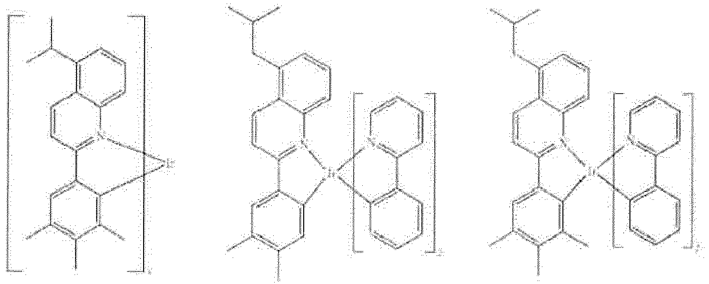

Further red emitters that may be used in the OLEDs according to the present invention are disclosed in US 2013/0146848 and are shown in the following:

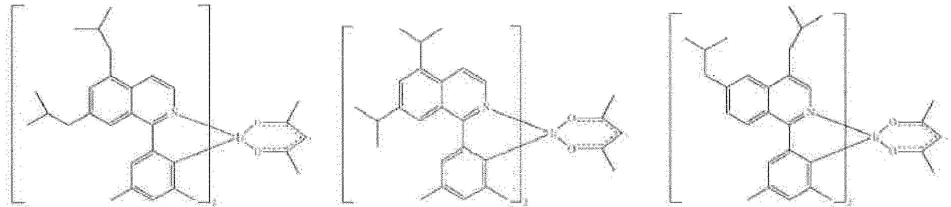

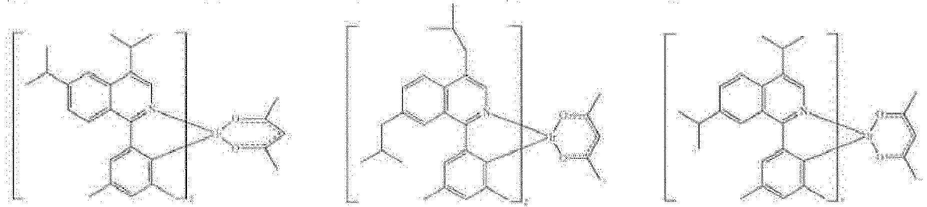

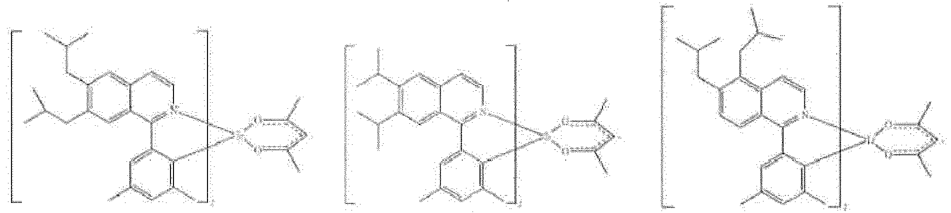

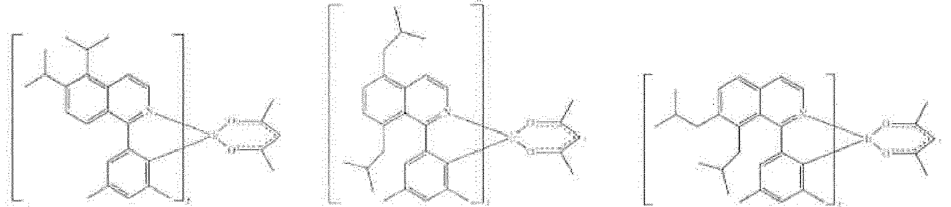

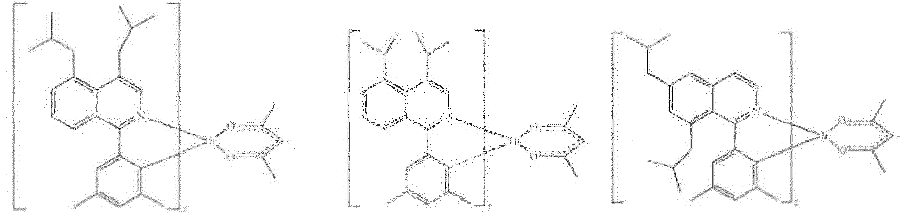

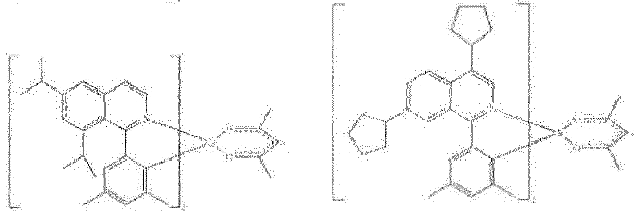

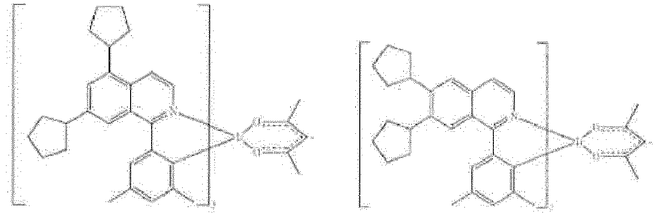

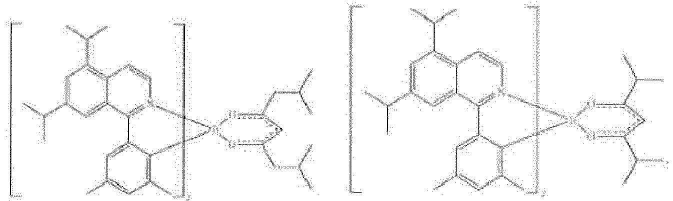

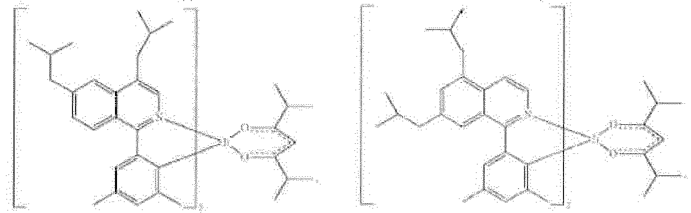

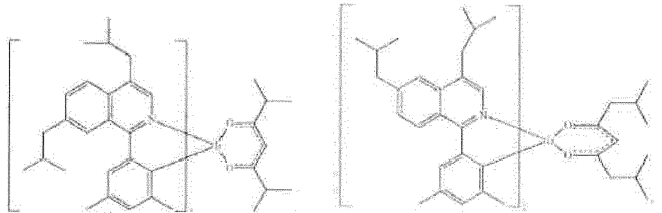

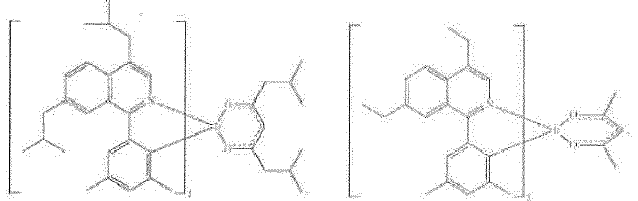

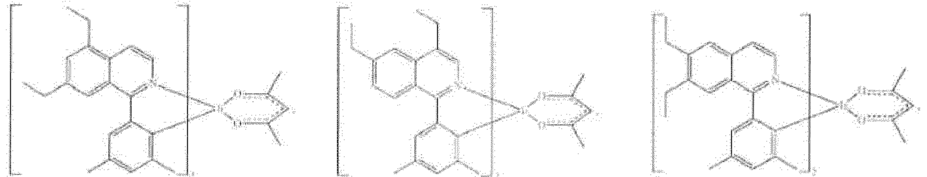

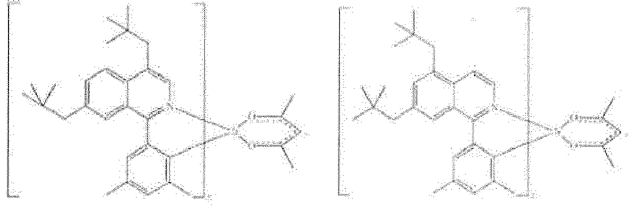

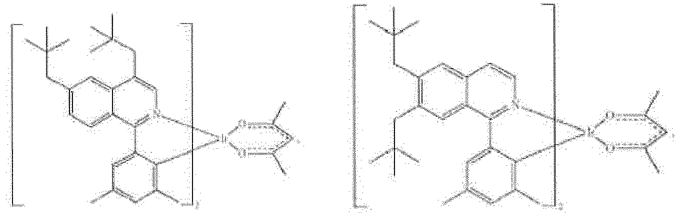

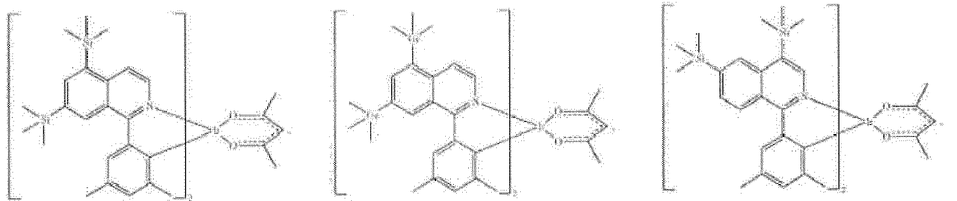

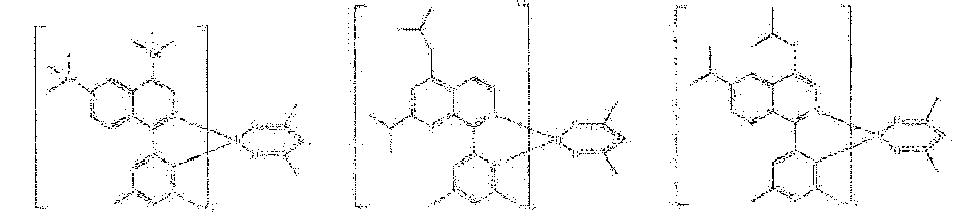

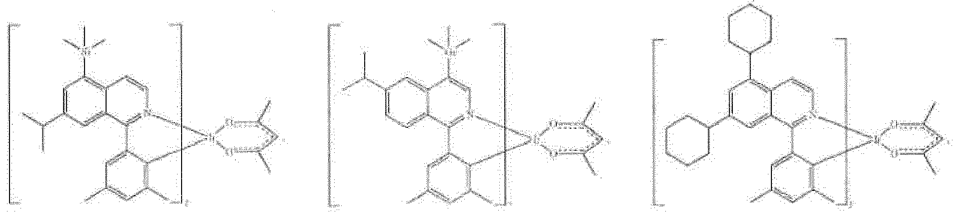

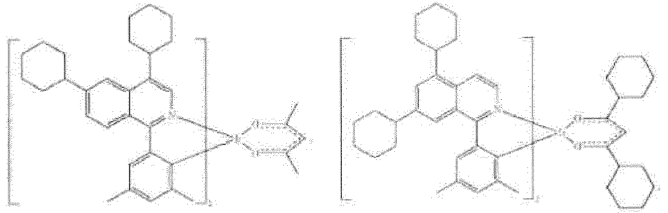

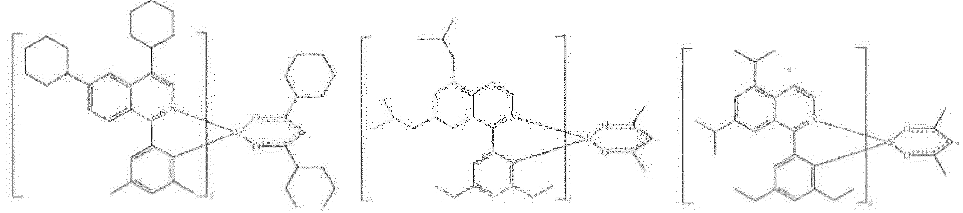

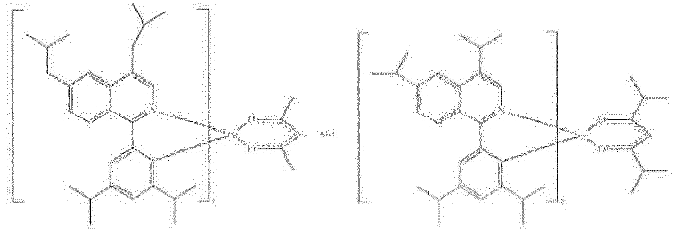

Further suitable red emitters are shown in US 2015/0001472. Preferred red emitters according to this document are the following compounds:

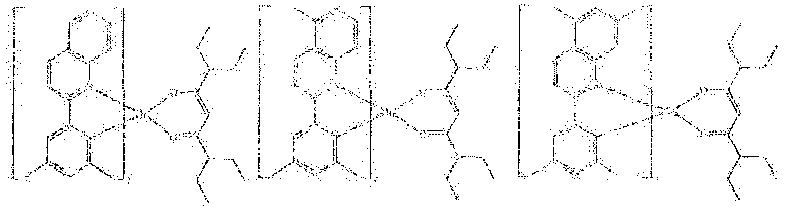

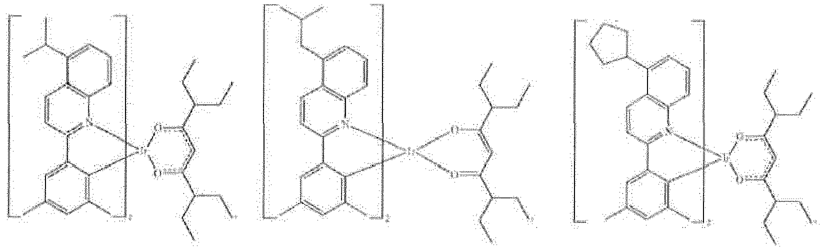

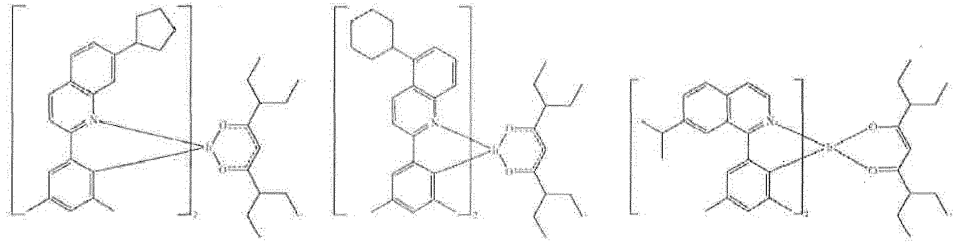

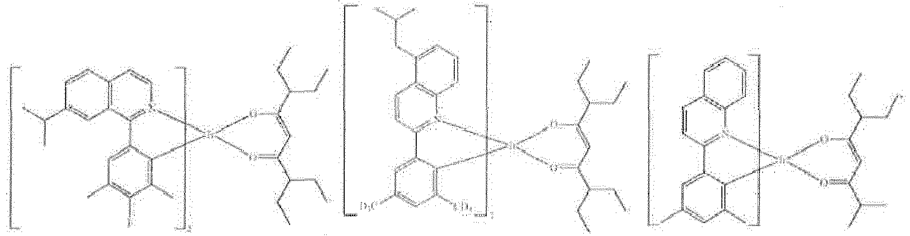

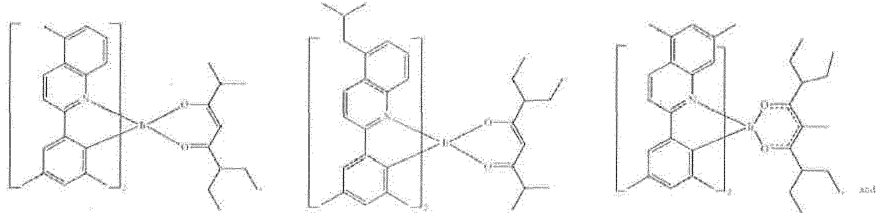

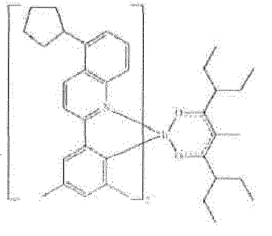

Further suitable red emitters are shown in US 2016/0093808. Preferred red emitters according to this document are the following compounds:

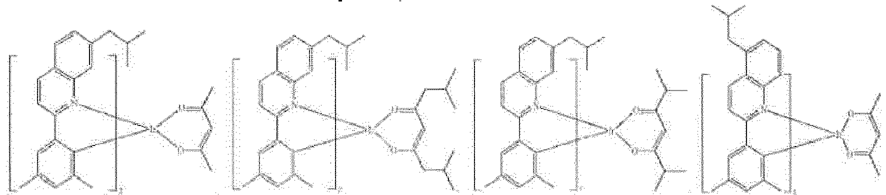

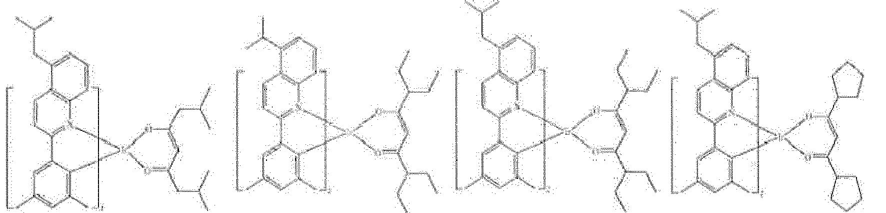

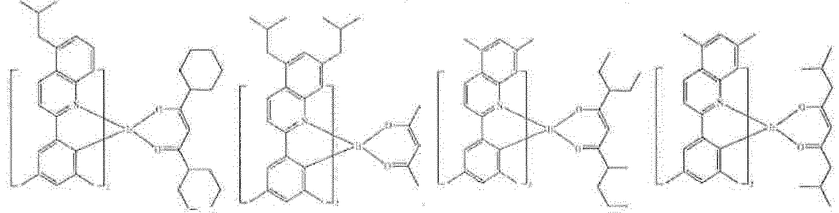

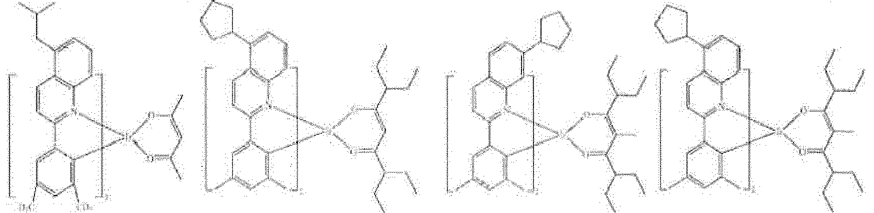

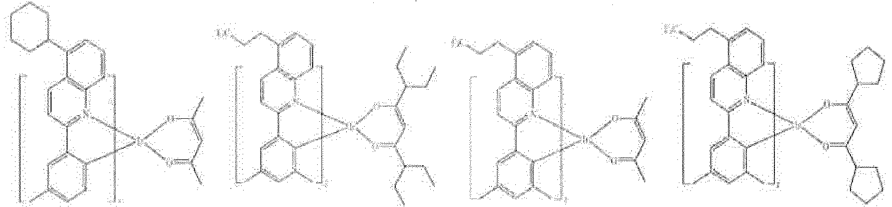

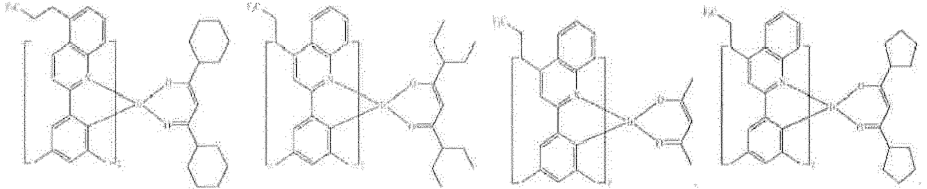

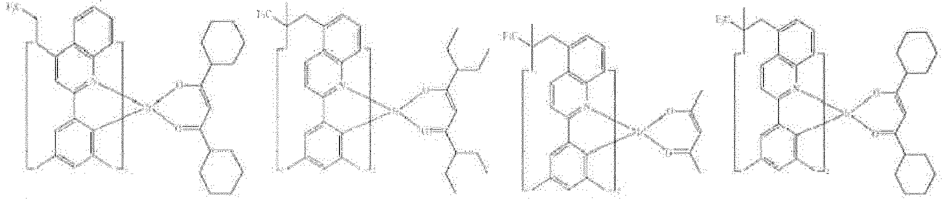

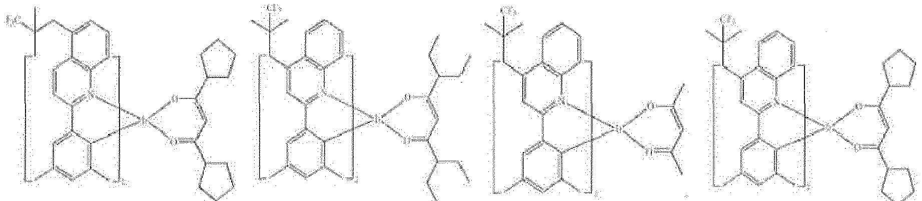

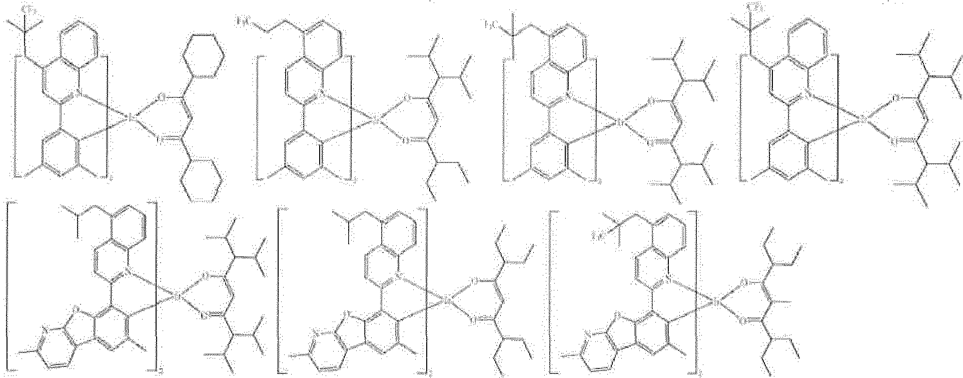

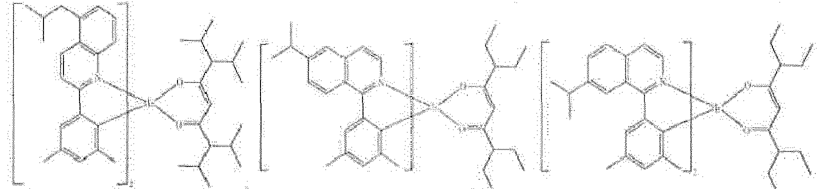

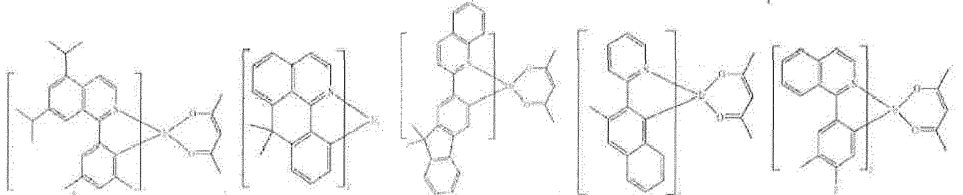

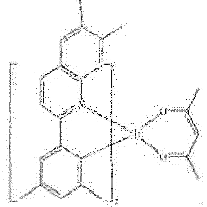

According to a further embodiment of the OLED according to the present invention, the light emitting layer may comprise at least one fluorescent, preferably blue, emitter. Examples of preferred blue dopants that may be present in the light emitting layer of the OLED according to the present invention are polycyclic amine derivatives as mentioned in EP 2924029. Particularly preferred aromatic amine derivatives are selected from compounds according to the following formula (20):

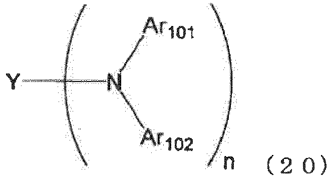

In the formula (20), Y is a substituted or unsubstituted fused aromatic hydrocarbon group including 10 to 50 ring carbon atoms.

Ar₁₀₁, and Ar₁₀₂ are independently a substituted or unsubstituted aryl group including 6 to 50 ring carbon atoms or a substituted or unsubstituted heterocyclic ring group including 5 to 50 ring atoms.

Specific examples of Y include the above-mentioned fused aryl group. Y is preferably a substituted or unsubstituted anthryl group, a substituted or unsubstituted pyrenyl group or a substituted or unsubstituted chrysenyl group.

n is an integer of 1 to 4. It is preferred that n be an integer of 1 to 2.

The above-mentioned formula (20) is preferably one represented by the following formulas (21) to (24).

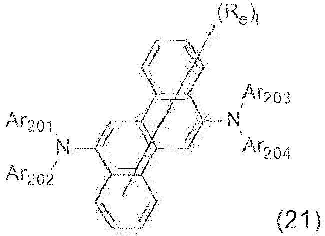

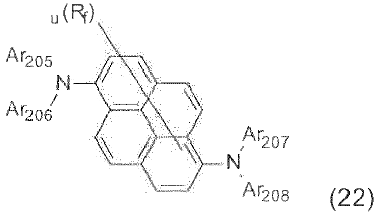

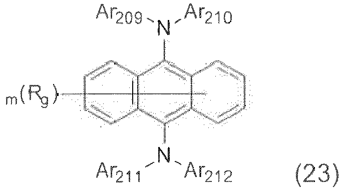

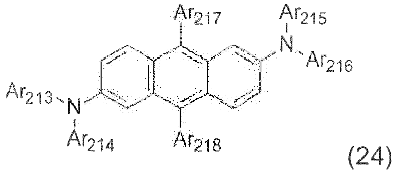

In the formulae (21) to (24), Rₑ, R_{f} and R_{g} are independently a substituted or unsubstituted alkyl group including 1 to 20 carbon atoms, a substituted or unsubstituted alkenyl group including 2 to 50 carbon atoms, a substituted or unsubstituted alkynyl group including 2 to 50 carbon atoms, a substituted or unsubstituted aralykyl group including 1 to 20 carbon atoms, a substituted or unsubstituted cycloalkyl group including 3 to 20 ring carbon atoms, a substituted or unsubstituted alkoxy group including 1 to 20 carbon atoms, a substituted or unsubstituted aryloxy group including 6 to 20 ring carbon atoms, a substituted or unsubstituted aryl group including 6 to 50 ring carbon atoms, a substituted or unsubstituted silyl group, a substituted or unsubstituted alkyl germanium group including 1 to 50 carbon atoms or a substituted or unsubstituted aryl germanium group including 6 to 50 ring carbon atoms. Rₑ, R_{f} and R_{g} may independently be bonded to any of the bonding positions of the benzene rings that constitutes the fused polycyclic skeleton.

As preferable examples of Rₑ, R_{f} and R_{g}, a substituted or unsubstituted aryl group including 6 to 50 ring carbon atoms can be given. More preferably, Rₑ, R_{f} and R_{g} are a substituted or unsubstituted phenyl group, a substituted or unsubstituted naphthyl group, or the like.

t is an integer of 0 to 10. u is an integer of 0 to 8. m is an integer of 0 to 10. Ar₂₀₁ to Ar₂₁₈ are independently an aryl group including 6 to 50 ring carbon atoms or a substituted or unsubstituted heterocyclic group including 5 to 50 ring atoms.

Preferred examples of Ar₂₀₁ to Ar₂₁₈ include a substituted or unsubstituted phenyl group, a substituted or unsubstituted dibenzofuranyl group or the like. As preferable examples of the substituent of Ar₂₀₁ to Ar₂₁₈, an alkyl group, a cyano group and a substituted or unsubstituted silyl group can be given.

In the formulae (21) to (24), as examples of the alkyl group, the alkoxy group, the aryl group, the aryloxy group and the heterocyclic group, those exemplified above can be given.

As the alkenyl group including 2 to 50, preferably 2 to 30, more preferably 2 to 20, and particularly preferably 2 to 10, carbon atoms, a vinyl group, an allyl group, a 1-butenyl group, a 2-butenyl group, a 3-butenyl group, a 1,3-butanedienyl group, a 1-methylvinyl group, a styryl group, a 2,2-diphenylvinyl group, a 1,2-diphenylvinyl group, a 1-methylallyl group, a 1,1-dimethylallyl group, a 2-methylallyl group, a 1-phenylallyl group, a 2-phenylallyl group, a 3-phenylallyl group, a 3,3-diphenylallyl group, a 1,2 - dimethylallyl group, a 1-phenyl-1-butenyl group, a 3-phenyl-1-butenyl group or the like can be given. Preferred are a styryl group, a 2,2-diphenylvinyl group, a 1,2-diphenylvinyl group or the like.

As the alkynyl group including 2 to 50 (preferably 2 to 30, more preferably 2 to 20, particularly preferably 2 to 10) carbon atoms, a propargyl group, a 3-pentynyl group or the like can be given.

As the alkyl germanium group, a methylhydrogermyl group, a trimethylgermyl group, a triethylgermyl group, a tripropylgermyl group, a dimethyl-t-butylgermyl group or the like can be given.

As the aryl germanium group, a phenyldihydrogermyl group, a diphenylhydrogermyl group, a triphenylgermyl group, a tritolylgermyl group, a trinaphthylgermyl group or the like can be given.

As the styrylamine compound and the styryldiamine compound, those represented by the following formulas (17) and (18) are preferable.

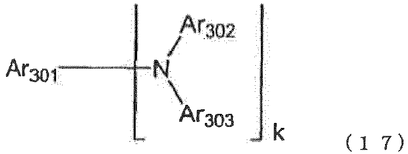

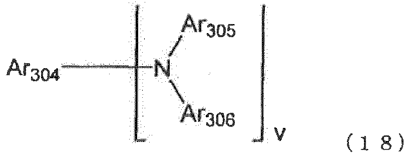

In the formula (17), Ar₃₀₁ is a k-valent group; a k-valent group corresponding to a phenyl group, a naphthyl group, a biphenyl group, a terphenyl group, a stilbene group, a styrylaryl group and a distyrylaryl group. Ar₃₀₂ and Ar₃₀₃ are independently an aryl group including 6 to 20 ring carbon atoms, and Ar₃₀₁, Ar₃₀₂ and Ar₃₀₃ may be substituted.

k is an integer of 1 to 4, with an integer of 1 and 2 being preferable. Any one of Ar₃₀₁ to Ar₃₀₃ is a group including a styryl group. It is further preferred that at least one of Ar₃₀₂ and Ar₃₀₃ be substituted by a styryl group.

As for the aryl group including 6 to 20 ring carbon atoms, the above-mentioned aryl group can be specifically given. Preferable examples include a phenyl group, a naphthyl group, an anthranyl group, a phenanthryl group, a terphenyl group or the like.

In the formula (18), Ar₃₀₄ to Ar₃₀₆ are a v-valent substituted or unsubstituted aryl group including 6 to 40 ring carbon atoms. v is an integer of 1 to 4, with an integer of 1 and 2 being preferable. Here, as the aryl group including 6 to 40 ring carbon atoms in the formula (18), the above-mentioned aryl group can be specifically given. A naphthyl group, an anthranyl group, a chrysenyl group, a pyrenyl group or an aryl group represented by the formula (20) is preferable.

As preferable substituents that substitute on the aryl group, an alkyl group including 1 to 6 carbon atoms, an alkoxy group including 1 to 6 carbon atoms, an aryl group including 6 to 40 ring carbon atoms, an amino group substituted by an aryl group including 6 to 40 ring carbon atoms, an ester group including an aryl group that includes 5 to 40 ring carbon atoms, an ester group including an alkyl group that includes 1 to 6 carbon atoms, a cyano group, a nitro group, a halogen atom or the like can be given.

The content of the emitter materials (dopants), preferably the phosphorescent emitter materials, in the light emitting layer is not particularly limited and selected according to the use of the device, and preferably 0.1 to 70% by mass, and more preferably 1 to 30% by mass. If being 0.1% by mass or more, the amount of light emission is sufficient. If being 70% by mass or less, the concentration quenching can be avoided. The further component in the emitting layer is usually one or more host material, which is preferably present in an amount of 30 to 99.9 % by mass, more preferably 70 to 99% by mass, wherein the sum of the emitter material(s) and the host material(s) is 100% by mass.

Further possible fluorescent blue emitters that may be present in the emitting layer of the OLED according to the present invention are mentioned in US2012112169.

### Host (matrix) materials

The compound of formula (I) is preferably employed as host material, more preferably as phosphorescent host material.

The light-emitting layer may comprise further components in addition to the emitter material. For example, a fluorescent dye may be present in the light-emitting layer in order to alter the emission color of the emitter material. In addition - in a preferred embodiment - a matrix material can be used. This matrix material may be a polymer, for example poly(N-vinylcarbazole) or polysilane. The matrix material may, however, be a small molecule, for example 4,4'-N,N'-dicarbazolebiphenyl (CDP=CBP) or tertiary aromatic amines, for example TCTA.

In the case that one or more phosphorescent emitter materials are used in the light emitting layer, one or more phosphorescent hosts are usually employed as host material. The phosphorescent host is a compound which confines the triplet energy of the phosphorescent dopant efficiently in the light emitting layer to cause the phosphorescent dopant to emit light efficiently.

In a preferred embodiment, the light-emitting layer is formed of at least one emitter material and of at least one of the matrix materials (hosts) mentioned in this application. According to a preferred embodiment, the electronic device according to the present invention, preferably the OLED according to the present invention, comprises at least one compound according to general formula (I) as matrix (host) material.

According to one embodiment, the light-emitting layer comprises at least one emitter material and at least two matrix materials (co-host system), wherein one of the matrix materials is a compound according to general formula (I) and the other matrix material(s) is/are used as co-host(s). Suitable other host materials than the compounds of general formula (I) (co-hosts) are known by a person skilled in the art. "Further host materials" means in the sense of the present application, host materials different from the compounds of general formula (I). However, it is also possible to use two or more different compounds of general formula (I) as host material in the light-emitting layer in an OLED of the present application. This embodiment is preferably realized with emitter materials that emit red light.

According to another embodiment, the light-emitting layer comprises at least one emitter material and a compound according to general formula (I) as a single matrix material. Examples of preferred compounds of general formula (I) useful as single host material are shown above. This embodiment is preferably realized with emitter materials that emit red light.

In a more preferred embodiment, the light-emitting layer is formed from 0.1 to 70% by weight, preferably 1 to 30% by weight, of at least one of the aforementioned emitter materials and 30 to 99.9% by weight, preferably 70 to 99% by weight, of at least one of the matrix materials mentioned in the specification - in one preferred embodiment at least one compound according to general formula (I) - where the sum total of the emitter material and of the matrix material adds up to 100% by weight.

In a further more preferred embodiment, the light-emitting layer comprises a compound of general formula (I) as matrix material, at least one further matrix material (co-host) and at least one emitter material. In said embodiment, the light-emitting layer is formed from 0.1 to 70% by weight, preferably 1 to 30% by weight, of the at least one emitter material and 30 to 99.9% by weight, preferably 70 to 99% by weight, of a compound according to general formula (I) and the further matrix material, where the sum total of the at least one emitter material, the further matrix material and of the compound of general formula (I) adds up to 100% by weight.

The content ratio of the compound according to general formula (I) as first host material and the second matrix material as co-host in the light emitting layer is not particularly limited and may be selected accordingly, and the ratio of first host material: second host material is preferably 1:99 to 99:1, more preferably 10:90 to 90:10, each based on mass.

Suitable host materials that may be used in the electronic device according to the present invention as host materials, if the compounds according to the present invention are not used as host material, but for example as charge transporting material, i.e. as electron transporting material or hole transporting material, are also known by a person skilled in the art.

According to the present invention, the compounds according to general formula (I) are preferably be used as host material in the light emitting layer of the electronic device, preferably in a OLED, according to the present invention. The compounds according to general formula (I) can be used (a) as single host materials or can be used (b) in combination with any compounds suitable as host materials as mentioned above.

### Electron-transporting layer

The electron-transporting layer is an organic layer that is formed between the emitting layer and the cathode and has a function of transporting electrons from the cathode to the emitting layer. When the electron-transporting layer is formed of plural layers, an organic layer that is nearer to the cathode is often defined as the electron-injecting layer. The electron-injecting layer has a function of injecting electrons from the cathode efficiently to the organic layer unit.
According to one embodiment, it is preferred that ET layer further comprising the other one or more layer(s) than electron injection layer to enhance efficiency and lifetime of the device, preferably between an electron injection layer and an emitting layer as a hole blocking layer, a exciton blocking layer or a triplet blocking layer.
A compound of the formula (1) is also preferable as all the use of the electron transporting layer, such as an electron transporting layer, an electron-injecting layer, a hole blocking layer, a exciton blocking layer or a triplet blocking layer.

According to one embodiment, it is preferred that an electron-donating dopant be contained in the interfacial region between the cathode and the emitting unit. Due to such a configuration, the organic EL device can have an increased luminance or a long life. Here, the electron-donating dopant means one having a metal with a work function of 3.8 eV or less. As specific examples thereof, at least one selected from an alkali metal, an alkali metal complex, an alkali metal compound, an alkaline earth metal, an alkaline earth metal complex, an alkaline earth metal compound, a rare earth metal, a rare earth metal complex and a rare earth metal compound or the like can be mentioned.

As the alkali metal, Na (work function: 2.36 eV), K (work function: 2.28 eV), Rb (work function: 2.16 eV), Cs (work function: 1.95 eV) and the like can be given. One having a work function of 2.9 eV or less is particularly preferable. Among them, K, Rb and Cs are preferable. Rb or Cs is further preferable. Cs is most preferable. As the alkaline earth metal, Ca (work function: 2.9 eV), Sr (work function: 2.0 eV to 2.5 eV), Ba (work function: 2.52 eV) and the like can be given. One having a work function of 2.9 eV or less is particularly preferable. As the rare-earth metal, Sc, Y, Ce, Tb, Yb and the like can be given. One having a work function of 2.9 eV or less is particularly preferable.

Examples of the alkali metal compound include an alkali oxide such as Li2O, Cs2O or K2O, and an alkali halide such as LiF, NaF, CsF and KF. Among them, LiF, Li2O and NaF are preferable. Examples of the alkaline earth metal compound include BaO, SrO, CaO, and mixtures thereof such as BaxSr1-xO (0<x<1) and BaxCa1-xO (0<x<1). Among them, BaO, SrO and CaO are preferable. Examples of the rare earth metal compound include YbF3, ScF3, ScO3, Y2O3, Ce2O3, GdF3 and TbF3. Among these, YbF3, ScF3 and TbF3 are preferable.

The alkali metal complexes, the alkaline earth metal complexes and the rare earth metal complexes are not particularly limited as long as they contain, as a metal ion, at least one of alkali metal ions, alkaline earth metal ions, and rare earth metal ions. Meanwhile, preferred examples of the ligand include, but are not limited to, quinolinol, benzoquinolinol, acridinol, phenanthridinol, hydroxyphenyloxazole, hydroxyphenylthiazole, hydroxydiaryloxadiazole, hydroxydiarylthiadiazole, hydroxyphenylpyridine, hydroxyphenylbenzimidazole, hydroxybenzotriazole, hydroxyfluborane, bipyridyl, phenanthroline, phthalocyanine, porphyrin, cyclopentadiene, β-diketones, azomethines, and derivatives thereof.

Regarding the addition form of the electron-donating dopant, it is preferred that the electron-donating dopant be formed in a shape of a layer or an island in the interfacial region. A preferred method for the formation is a method in which an organic compound (a light emitting material or an electron-injecting material) for forming the interfacial region is deposited simultaneously with deposition of the electron-donating dopant by a resistant heating deposition method, thereby dispersing the electron-donating dopant in the organic compound. The dispersion concentration of the organic compound: the electron-donating dopant (molar ratio) is 100:1 to 1:100, preferably 5:1 to 1:5.

In a case where the electron-donating dopant is formed into the shape of a layer, the light-emitting material or electron-injecting material which serves as an organic layer in the interface is formed into the shape of a layer. After that, a reductive dopant is solely deposited by the resistant heating deposition method to form a layer preferably having a thickness of from 0.1 nm to 15 nm. In a case where the electron-donating dopant is formed into the shape of an island, the emitting material or the electron-injecting material which serves as an organic layer in the interface is formed into the shape of an island. After that, the electron-donating dopant is solely deposited by the resistant heating deposition method to form an island preferably having a thickness of from 0.05 nm to 1 nm.

The ratio of the main component and the electron-donating dopant in the organic EL device according to the invention is main component: electron-donating dopant = 5 : 1 to 1 : 5 in terms of molar ratio, more preferably 2 : 1 to 1 : 2.

As the electron-transporting material used in the electron-transporting layer other than a compound of the formula (1), an aromatic heterocyclic compound having one or more hetero atoms in the molecule may preferably be used. In particular, a nitrogen containing heterocyclic derivative is preferable.
According to one embodiment, it is preferable that ET layer comprises a nitrogen containing heterocyclics metal chelate, such as 8-hydroxyquinolinolato aluminum, which is generally called as Alq₃.

According to the other embodiment, it is preferable that ET layer comprising substituted or unsubstituted nitrogen containing heterocyclic derivative.
Specific examples of the preferable heterocyclic derivative for ET layer are, 6-membered azine derivatives; such as pyridine derivatives, pyrimidine derivatives, triazine derivatives, pyrazine derivatives, preferably pyrimidine derivatives or triazine derivatives; 6-membered fused azine derivatives, such as quinolone derivatives, isoquinoline derivatives, quinoxaline derivatives, quinazoline derivatives, phenanthroline derivatives, benzoquinoline derivatives, benzoisoquinoline derivatives, dibenzoquinoxaline derivatives, preferably quinolone derivatives, isoquinoline derivatives, phenanthroline derivatives; 5-membered heterocyclic derivatives, such as imidazole derivatives, oxazole derivatives, oxadiazole derivatives, triazole derivatives, thiazole derivatives, thiadiazole derivatives; fused imidazole derivatives, such as benzimidazole derivatives, imidazopyridine derivatives, naphthoimidazole derivatives, benzimidazophenanthridine derivatives, benzimidzobenzimidazole derivatives, preferably benzimidazole derivatives, imidazopyridine derivatives or benzimidazophenanthridine derivatives.
According to the other embodiment, it is preferable ET layer comprises phosphine oxide derivative represented as Arₚ₁Arₚ₂Ar_{P3}P=O.
Arₚ₁∼Arₚ₃ are the substituents of phosphor atom and each independently represent substituted or unsubstituted above mentioned aryl group or substituted or unsubstituted above mentioned heterocyclic group.
According to the other embodiment, it is preferable that ET layer comprises aromatic hydrocarbon derivatives.
Specific examples of the preferable aromatic hydrocarbon derivatives for ET layer are, oligophenylene derivatives, naphthalene derivatives, fluorene derivatives, fluoranthenyl group, anthracene derivatives, phenanthrene derivatives, pyrene derivatives, triphenylene derivatives, benzanthracene derivatives, chrysene derivatives, benzphenanthrene derivatives, naphthacene derivstives, benzochrysene derivatives, and so on, preferably anthracene derivatives, pyrene derivatives and fluoranthene derivatives.

### Cathode

The cathode (i) is an electrode which serves to introduce electrons or negative charge carriers. The cathode may be any metal or nonmetal which has a lower work function than the anode. Suitable materials for the cathode are selected from the group consisting of alkali metals of group 1, for example Li, Cs, alkaline earth metals of group 2, metals of group 12 of the Periodic Table of the Elements, comprising the rare earth metals and the lanthanides and actinides. In addition, metals such as aluminum, indium, calcium, barium, samarium and magnesium, and combinations thereof, may be used.

In general, the different layers, if present, have the following thicknesses:
anode (a): 500 to 5000 Å (ångström), preferably 1000 to 2000 Å;
hole injection layer (b): 50 to 1000 Å, preferably 200 to 800 Å,
hole-transport layer (c): 50 to 1000 Å, preferably 100 to 800 Å,
exciton blocking layer (d): 10 to 500 Å, preferably 50 to 100 Å,
light-emitting layer (e): 10 to 1000 Å, preferably 50 to 600 Å,
hole/ exciton blocking layer (f): 10 to 500 Å, preferably 50 to 100 Å,
electron-transport layer (g): 50 to 1000 Å, preferably 200 to 800 Å,
electron injection layer (h): 10 to 500 Å, preferably 20 to 100 Å,
cathode (i): 200 to 10 000 Å, preferably 300 to 5000 Å.

The person skilled in the art is aware (for example on the basis of electrochemical studies) of how suitable materials have to be selected. Suitable materials for the individual layers are known to those skilled in the art and are disclosed, for example, in WO 00/70655.

In addition, it is possible that some of the layers used in the inventive OLED have been surface-treated in order to increase the efficiency of charge carrier transport. The selection of the materials for each of the layers mentioned is preferably determined by obtaining an OLED with a high efficiency and lifetime.

The inventive electronic device, preferably OLED, can be produced by methods known to those skilled in the art. In general, the inventive OLED is produced by successive vapor deposition of the individual layers onto a suitable substrate. Suitable substrates are, for example, glass, inorganic semiconductors or polymer films. For vapor deposition, it is possible to use customary techniques, such as thermal evaporation, chemical vapor deposition (CVD), physical vapor deposition (PVD) and others. In an alternative process, the organic layers of the OLED can be applied from solutions or dispersions in suitable solvents, employing coating techniques known to those skilled in the art.

Use of the compounds according to general formula (I) in at least one layer of the OLED, preferably in the light-emitting layer, preferably as a host material, a charge transporting material, particularly preferably as a host material and hole or electron transporting material, makes it possible to obtain OLEDs, with high efficiency and with low use and operating voltage. Frequently, the OLEDs obtained by the use of the compounds according to general formula (I) additionally have long lifetime. The efficiency of the electronic devices, preferably OLEDs can additionally be improved by optimizing the other layers of the OLEDs. For example, high-efficiency cathodes such as Ca or Ba, if appropriate in combination with an intermediate layer of LiF, can be used. Moreover, additional layers may be present in the OLEDs in order to adjust the energy level of the different layers and to facilitate electroluminescence.

The OLEDs may further comprise at least one second light-emitting layer. The overall emission of the OLEDs may be composed of the emission of the at least two light-emitting layers and may also comprise white light.

The OLEDs can be used in all apparatus in which electroluminescence is useful. Suitable devices are preferably selected from stationary and mobile visual display units and illumination units. Stationary visual display units are, for example, visual display units of computers, televisions, visual display units in printers, kitchen appliances and advertising panels, illuminations and information panels. Mobile visual display units are, for example, visual display units in cellphones, tablet PCs, laptops, digital cameras, MP3 players, vehicles and destination displays on buses and trains. Further devices in which the inventive OLEDs can be used are, for example, keyboards; items of clothing; furniture; wallpaper. In addition, the present invention relates to a device selected from the group consisting of stationary visual display units such as visual display units of computers, televisions, visual display units in printers, kitchen appliances and advertising panels, illuminations, information panels, and mobile visual display units such as visual display units in cellphones, tablet PCs, laptops, digital cameras, MP3 players, vehicles and destination displays on buses and trains; illumination units; keyboards; items of clothing; furniture; wallpaper, comprising at least one inventive organic light-emitting diode or at least one inventive light-emitting layer.

The following examples are included for illustrative purposes only and do not limit the scope of the claims. Unless otherwise stated, all parts and percentages are by weight.

### Examples

### I Synthesis Examples

### Examples

### Synthetic pathway

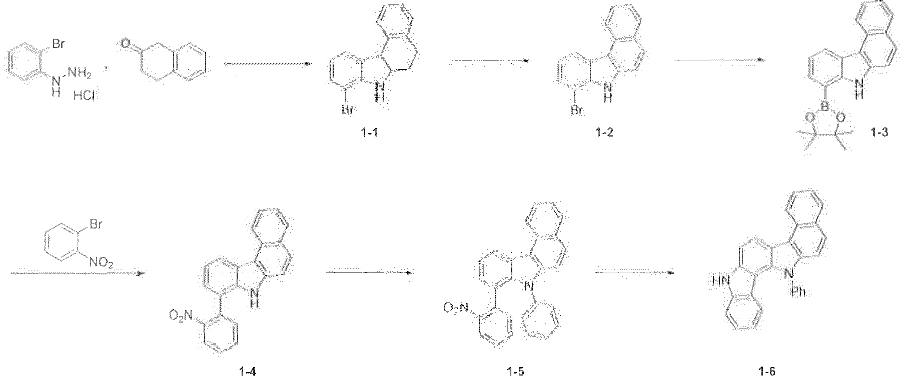

### Intermediate 1-1

(2-Bromophenyl)hydrazine HCI salt (7.48 g, 40.0 mmol) was suspended in water (20 mL) and HCI (21 mL, 4 M in dioxane) in a 100 mL three-necked round-bottom flask. The flask was fitted with a reflux condenser and heated to 85 °C, where 2-tetralone (5.55 mL, 42.0 mmol) was added dropwise over 10 min. The reaction was refluxed for 16 h. Then, the reaction was allowed to cool to room temperature and neutralized with 30% aqueous NaOH solution. The layers were transferred to a 250 mL separation funnel, separated and the aqueous layer was extracted with EtOAc (3x40 mL). The combined organic layers were dried (Na₂SO₄), filtered and concentrated. The residue was purified via flash chromatography (EtOAc/heptane) to give 6.37 g (54%) of an off-white solid. The molecular mass of the product was confirmed by LC-MS [M+H] 296.

### Intermediate 1-2

To a 250 mL three-necked round-bottom flask were added intermediate 1-1 (6.3 g, 21.1 mmol), p-chloranil (5.45 g, 22.2 mmol) and degassed xylenes (75 mL). The reaction was fitted with a reflux condenser, placed under Ar and heated to reflux for 16 h. Then, the reaction was allowed to cool to room temperature and the solid was filtered off. The resulting filtrate was concentrated, then purified via flash chromatography (EtOAc/heptane) to give 5.9 g (95%) of a grey solid. The molecular mass of the product was confirmed by LC-MS [M+H] 294.

### Intermediate 1-3

To a dried 250 mL three-necked round-bottom flask were added intermediate 1-2 (12 g, 40.5 mmol), bis(pinacolato)diboron (13.38 g, 52.7 mmol), potassium acetate (7.95 g, 81 mmol) and Pd(dppf)Cl₂ (1.20 g, 1.621 mmol). The reaction was evacuated and backfilled with Ar (x3), then degassed dioxane (101 mL) was added. The reaction was gently refluxed at 100 °C for 15 h. Then, the reaction was allowed to cool down to room temperature and filtered over a pad of silica/celite. The filtrate was washed with water (100 mL), dried (Na₂SO₄), filtered and concentrated. The resulting residue was used as is for the next step.

### Intermediate 1-4

To a 250 mL three-necked round-bottom flask were added intermediate 1-3 (10.91 g, 31.8 mmol), 1-bromo-2-nitrobenzene (7.71 g, 38.1 mmol), NaOH (3.81 g, 95 mmol) and Pd(PPh₃)₄ (1.102 g, 0.954 mmol). The flask was purged with N₂ for 5 min, then THF (70.6 mL) and water (35.3 mL) were added. The reaction was fitted with a reflux condenser and gently refluxed for 16 h. Then, the reaction was allowed to cool down to room temperature and transferred to a 500 mL separation funnel. The aqueous layer was extracted with EtOAc (3x100 mL). The combined organic layers were dried (Na₂SO₄), filtered and concentrated. The residue was purified via flash chromatography (EtOAc/heptane) to give 9.36 g (87%) of an off-white solid. The molecular mass of the product was confirmed by LC-MS [M+H] 337.0.

### Intermediate 1-5

To a dried 250 mL three-necked round-bottom flask were added intermediate 1-4 (9 g, 26.6 mmol), potassium carbonate (11.03 g, 80 mmol) and copper (1.69 g, 26.6 mmol). The flask was fitted with a reflux condenser and placed under N₂. Nitrobenzene (26.6 ml) and iodobenzene (7.5 mL, 66.9 mmol) were added and the reaction was heated to 190 °C for 14 h. Then, the reaction was allowed to cool down to room temperature, diluted with EtOAc (100 mL) and transferred to a 500 mL separation funnel. The organic layer was thoroughly washed with a 1% solution of 2-aminopropanol in water. The organic layer was dried (Na₂SO₄), filtered and concentrated. The resulting solid was dried, then recrystallized from toluene. After filtration, 6.93 g (63%) yellow solid were obtained. The molecular mass of the product was confirmed by LC-MS [M+H] 415.3.

### Intermediate 1-6

To a dried 100 mL three-necked round-bottom flask were added intermediate 1-5 (6.9 g, 16.65 mmol) and triphenylphosphine (21.83 g, 83 mmol). The reaction was placed under N₂, and heated at 200 °C for 16 h. Then, the reaction was allowed to cool down to room temperature, diluted with heptanes and filtered. The resulting solid was washed several times with refluxing methanol, then further purified by a hot filtration with toluene and chlorobenzene. 5 g (79%) of a grey solid were obtained. The molecular mass of the product was confirmed by LC-MS [M+H] 383.3.

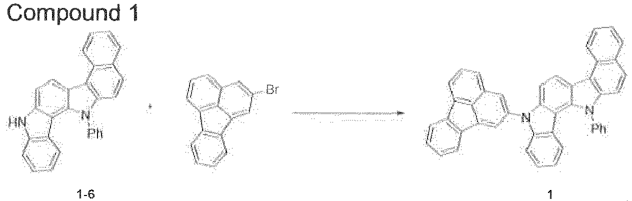

To a 100 mL Schlenk flask were added intermediate 1-6 (1.8 g, 4.71 mmol), 2-bromofluoranthene (1.43 g, 5.08 mmol), sodium *tert*-butoxide (0.63 g, 6.6 mmol), Pd₂dba₃ (0.086 g, 0.094 mmol) and P*t*Bu₃HBF₄ (0.109 g, 0.377 mmol). The flask was purged with N₂ for 5 min, then toluene (26 mL) was added. The reaction was stirred at 100 °C for 15 h. Then, the reaction was cooled to room temperature, and the resulting solid was filtered, rinsing with EtOH (15 mL). The solid was purified via flash chromatography (DCM/heptane) to give 1.64 g (60%) of a yellow solid. The molecular mass of the product was confirmed by LC-MS [M+H] 583.4.

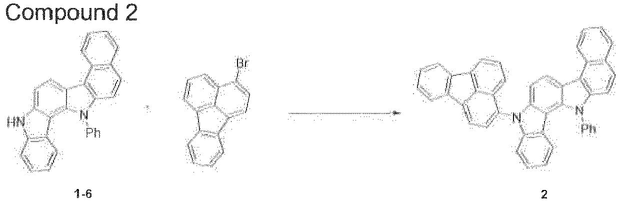

Compound 2 was synthesized via a similar procedure to compound 1, except that 3-bromofluoranthene was used as the coupling partner. After flash chromatography, 1.38 g (50%) yellow solid were obtained. The molecular mass of the product was confirmed by LC-MS [M+H] 583.2.

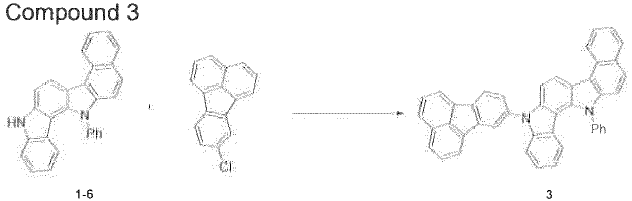

Compound 3 was synthesized via a similar procedure to compound 1, except that 8-chlorofluoranthene was used as the coupling partner and the reaction was done on a 3.14 mmol scale. After flash chromatography, 1.51 g (83%) yellow solid was obtained. The molecular mass of the product was confirmed by LC-MS [M+H] 583.4.

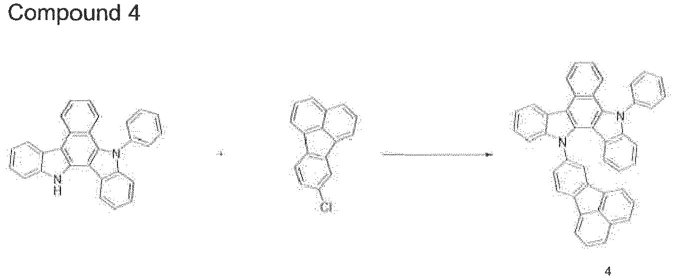

To a 100 mL three-necked round-bottom flask were added 5-phenyl-5,14-dihydrobenzo[a]indo-lo[3,2-c]carbazole (1.91 g, 5.00 mmol), 8-chlorofluoranthene (1.30 g, 5.50 mmol), sodium *tert-*butoxide (0.72 g, 7.50 mmol), Pd₂dba₃ (0.114 g, 0.125 mmol) and P*t*Bu₃HBF₄ (0.145 g, 0.50 mmol). The flask was purged with N₂ for 5 min, then xylenes (28 mL) was added. The reaction was stirred at 110 °C for 18 h. Then, the reaction was cooled to room temperature, and the resulting solid was filtered, rinsing with EtOH (15 mL). The solid was purified via flash chromatography (DCM/heptane) to give 2.77 g (95%) of a yellow solid. The molecular mass of the product was confirmed by LC-MS [M+H] 583.4.

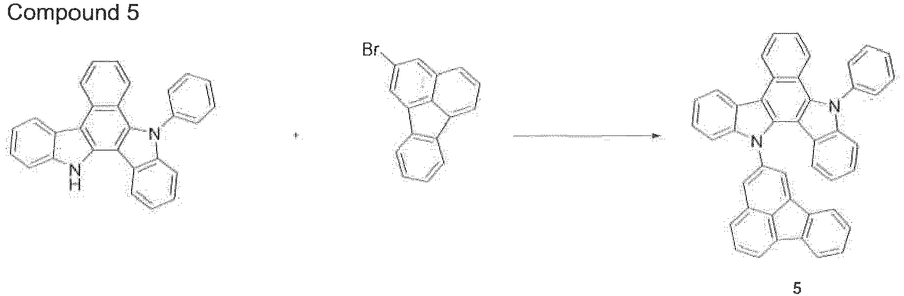

To a 100 mL three-necked round-bottom flask were added 5-phenyl-5,14-dihydrobenzo[a]indolo[3,2-c]carbazole (1.42 g, 3.71 mmol), 2-bromofluoranthene (1.04 g, 3.71 mmol), sodium *tert*-butoxide (0.50 g, 5.20 mmol), Pd₂dba₃ (0.114 g, 0.074 mmol) and *c*BRIDP (0.105 g, 0.30 mmol). The flask was purged with N₂ for 5 min, then toluene (15 mL) was added. The reaction was stirred at 110 °C for 15 h. Then, the reaction was cooled to room temperature, and the resulting solid was filtered, rinsing with EtOH (15 mL). The solid was purified via flash chromatography (toluene/heptane) to give 1.44 g (67%) of a yellow solid. The molecular mass of the product was confirmed by LC-MS [M+H] 583.3.

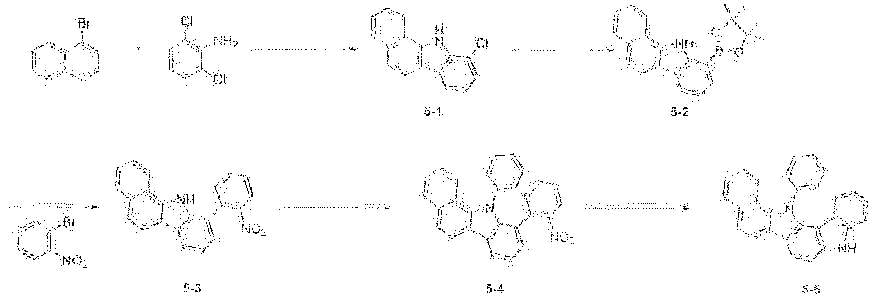

### Intermediate 5-1

To a 500 mL three-necked round-bottom flask were added 1-bromonaphthalene (10 g, 48.3 mmol), 2,6-dichloroaniline (7.82 g, 48.3 mmol), Pd(OAc)₂ (0.325 g, 1.45 mmol), P*t*Bu₃HBF₄ (0.84 g, 2.90 mmol), NaO*t*Bu (9.28 g, 97 mmol) and xylene (220 mL) under an N₂ atmosphere. The reaction was stirred at 100 °C for 1 h, then at 130 °C for 23 h. Then the reaction was cooled to room temperature, and the solvent was removed in vacuo. The resulting residue was purified via flash chromatography (toluene/heptane) to give 5.8 g (48%) of an off-white solid. The molecular mass of the product was confirmed by LC-MS [M-H] 250.0.

### Intermediates 5-2 and 5-3

To a 1 L three-necked round-bottom flask was added Intermediate 5-1 (16 g, 63.6 mmol), bis(pinacolato)diboron (16.14 g, 63.6 mmol), potassium acetate (9.36 g, 95 mmol), Pd₂(dba)₃ (0.582 g, 0.64 mmol), Sphos (2-Dicyclohexylphosphino-2',6'-dimethoxybiphenyl) (1.04 g, 2.54 mmol) and 1,4-dioxane (318 mL) under an N₂ atmosphere. The reaction was stirred at 105 °C for 18 h. Then the reaction was cooled to room temperature, transferred to a separatory funnel, where it was washed with water (150 mL). The aqueous layer was extracted with EtOAc (2 x 150 mL). The combined organic layers were dried (Na₂SO₄), filtered and concentrated in vacuo. The resulting Intermediate 5-2 was used as is for the next step.
To a 1 L flask were added Intermediate 5-2 from above, 1-bromo-2-nitrobenzene (12.36 g, 61.2 mmol), potassium phoshate (26.0 g, 122 mmol), Pd(PPh₃)₄ (1.41 g, 1.22 mmol) and THF/water (245/62 mL). The reaction was refluxed at 75 °C under an N₂ atmosphere for 19 h. Then, the reaction was cooled to room temperature, transferred to a separatory funnel where it was washed with water (150 mL). The aqueous layer was extracted with EtOAc (2 x 150 mL). The combined organic layers were dried (Na₂SO₄), filtered and concentrated in vacuo. The resulting residue was purified via flash chromatography (toluene/heptane) to give 18 g (84% over two steps) of Intermediate 5-3 as a yellow solid. The molecular mass of the product was confirmed by LC-MS [M-H] 337.3.

### Intermediate 5-4

To a 250 mL three-necked round-bottom flask were added Intermediate 5-3 (10 g, 29.6 mmol), iodobenzene (10.8 mL, 96.4 mmol), copper (1.88 g, 29.6 mmol), potassium carbonate (12.25 g, 89 mmol) and nitrobenzene (60 mL). The reaction was stirred at 195 °C under an atmosphere of N₂ for 48 h. Then, the reaction was allowed to cool down to room temperature, diluted with dichloromethane (100 mL) and transferred to a separation funnel. The organic layer was thoroughly washed with a 1% solution of 2-aminopropanol in water. The organic layer was dried (Na₂SO₄), filtered and concentrated. The resulting solid was dried, then recrystallized from toluene. After filtration, 8.5 g (69%) of a yellow solid were obtained. The molecular mass of the product was confirmed by LC-MS [M+H] 415.3.

### Intermediate 5-5

To a 100 mL three-necked round-bottom flask were added Intermediate 5-4 (5 g, 12.06 mmol) and triphenylphosphine (29.9 g, 114 mmol). The reaction was placed under N₂, and heated at 175 °C for 2 h. Then, the reaction was allowed to cool down to room temperature, and triturated with heptane (500 mL) to remove unreacted PPh₃. The heptane was decanted and the residue obtained was further purified via flash chromatography (toluene/heptane). The resulting solid was recrystallized from toluene to give 3 g (65%) of a white solid. The molecular mass of the product was confirmed by LC-MS [M-H] 381.2.

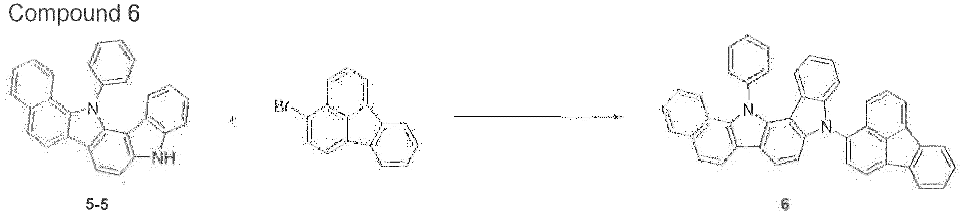

To a 100 mL three-necked round-bottom flask were added Intermediate 5-5 (1.2 g, 3.14 mmol), 3-bromofluoranthene (1.06 g, 3.77 mmol), sodium *tert*-butoxide (0.45 g, 4.71 mmol), Pd₂dba₃ (0.086 g, 0.094 mmol) and P*t*Bu₃HBF₄ (0.109 g, 0.38 mmol). The flask was purged with N₂ for 5 min, then xylene (21 mL) was added. The reaction was stirred at 130 °C for 24 h. Then, the reaction was cooled to room temperature, and transferred to a 250 mL separatory funnel, where it was washed with water (30 mL). The aqueous layer was extracted with dichloromethane (3 x 40 mL). The combined organic layers were dried (Na₂SO₄), filtered and concentrated. The residue was purified via flash chromatography (dichloromethane/heptane) to give 1.33 g (73%) of a yellow solid. The molecular mass of the product was confirmed by LC-MS [M+H] 583.3.

### Application Examples

### Comparative Application Example 1

A glass substrate with 120 nm-thick indium-tin-oxide (ITO) transparent electrode (manufactured by Geomatec Co., Ltd.) used as an anode was first cleaned with isopropanol in an ultrasonic bath for 10 min. To eliminate any possible organic residues, the substrate was exposed to an ultraviolet light and ozone for further 30 min. This treatment also improves the hole injection properties of the ITO. The cleaned substrate was mounted on a substrate holder and loaded into a vacuum chamber. Thereafter, the organic materials specified below were applied by vapor deposition to the ITO substrate at a rate of approx. 0.2-1 Å/sec at about 10⁻⁶-10⁻⁸ mbar. As a hole injection layer, 5 nm-thick of compound HI was applied, Then 220 nm-thick of compound HT1 was applied as hole transporting layer 1. Subsequently, a mixture of 2% by weight of an emitter compound (EM), 98% by weight of a host (Comparative compound 1) were applied to form a 40 nm-thick phosphorescent-emitting layer. On the emitting layer, 30 nm-thick layer of coevaporated compound ET and Liq at ratio 1:1 by weight is applied as an electron transport layer. Finally, 1 nm-thick Liq is deposited as an electron injection layer and 80 nm-thick Al is then deposited as a cathode to complete the device. The device was sealed with a glass lid and a getter in an inert nitrogen atmosphere with less than 1 ppm of water and oxygen.

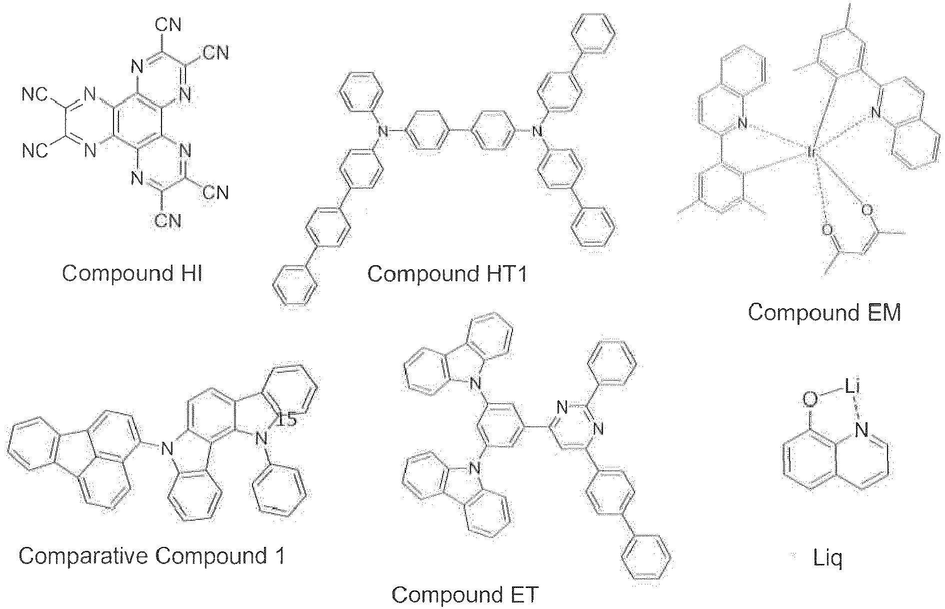

### Comparative Application Example 2

Comparative Application Example 1 was repeated except for using Comparative compound 2 in place of the host (Comparative compound 1). The device results were shown in Table 1.

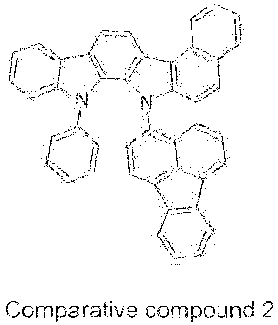

### Comparative Application Example 3

Comparative Application Example 1 was repeated except for using Comparative compound 3 in place of the host (Comparative compound 1). The device results were shown in Table 1.

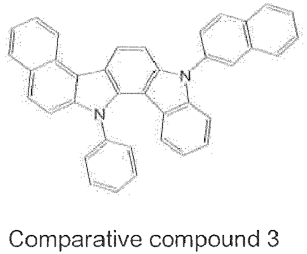

### Application Examples 1-3

Comparative Application Example 1 was repeated except for using each compound shown in Table 1 in place of the host (Comparative compound 1). The device results were shown in Table 1.

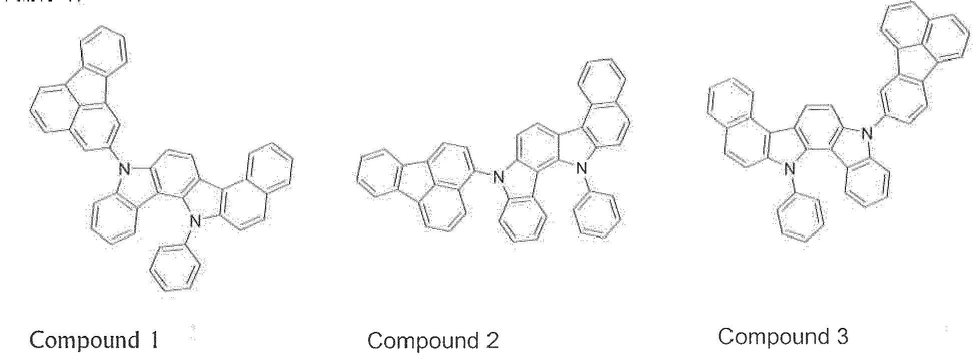

### OLED Characterization

To characterize the OLED, electroluminescence spectra were recorded at various currents and voltages. In addition, the current-voltage characteristic was measured in combination with the luminance to determine current efficiency. Current efficiency was given at a current density of 10mA/cm². Lifetime of devices was measured at constant current of 50mA/cm². LT95 represents the time it takes for the brightness of a device operated under a constant current of 50mA/cm² to drop by 5%. The device results were shown in Table 1.

**Table 1**

| Appl. Ex. | Host | Voltage, V | Current Efficiency, cd/A | LT95, h | CIE(x,y) |
|---|---|---|---|---|---|
| Comp. Appl. Ex. 1 | Comparative Compound 1 | 4.99 | 12.3 | 123 | 0.67, 0.33 |
| Comp. Appl. Ex. 2 | Comparative Compound 2 | 5.02 | 15.02 | 2.7 | 0.67, 0.33 |
| Comp. Appl. Ex. 3 | Comparative Compound 3 | 5.13 | 6.08 | 10.5 | 0.67, 0.32 |
| App. Ex. 1 | Compound 1 | 4.22 | 18.48 | 170 | 0.67, 0.33 |
| App. Ex. 2 | Compound 2 | 4.19 | 16.6 | 143 | 0.67, 0.33 |
| App. Ex. 3 | Compound 3 | 4.32 | 18.5 | 173 | 0.67, 0.33 |

The results shown in Table 1 demonstrate that the OLED performance (Voltage, current efficiency and lifetime) were improved in the case that an inventive compound 1, 2 and 3 were used as red hosts in an OLED.

### Comparative Application Example 4

Comparative Application Example 1 was repeated except for using Comparative compound 4 in place of the host (Comparative compound 1). The device results were shown in Table 2.

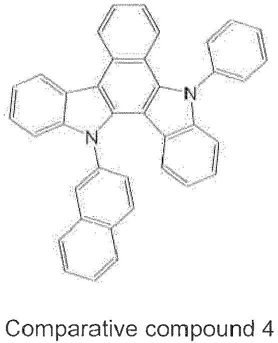

### Application Examples 4-6

Comparative Application Example 1 was repeated except for using each compound shown in Table 2 in place of the host (Comparative compound 1).

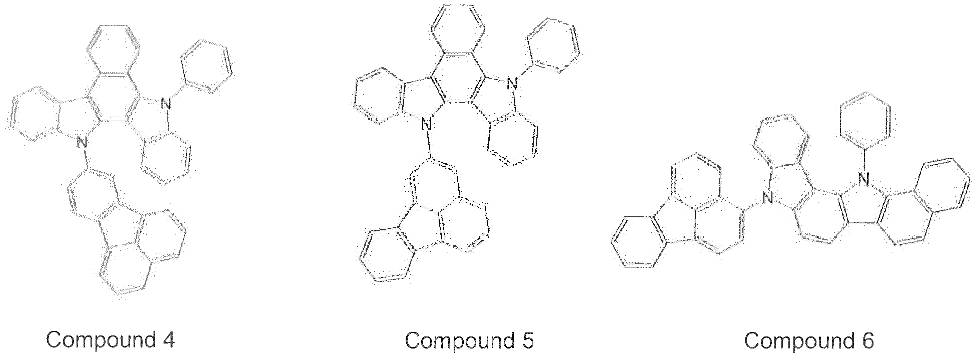

### OLED Characterization

To characterize the OLED, electroluminescence spectra were recorded at various currents and voltages. In addition, the current-voltage characteristic was measured in combination with the luminance to determine current efficiency. Current efficiency was given at a current density of 10mA/cm². The device results were shown in Table 2.

**Table 2**

| Appl. Ex. | Host | Voltage, V | Current Efficiency, cd/A | CIE(x,y) |
|---|---|---|---|---|
| Comp. Appl. Ex. 4 | Comparative Compound 4 | 4.84 | 4.91 | 0.67, 0.32 |
| App. Ex. 4 | Compound 4 | 4.27 | 14.9 | 0.68, 0.31 |
| App. Ex. 5 | Compound 5 | 4.25 | 17.57 | 0.66, 0.33 |
| App. Ex. 6 | Compound 6 | 3.94 | 16.58 | 0.68, 0.31 |

The results shown in Table 2 demonstrate that the OLED performance (Voltage and current efficiency) was improved in the case that an inventive compound 4, 5 and 6 were used as red hosts in an OLED.

## Claims

1. A polycyclic compound represented by formula (I): 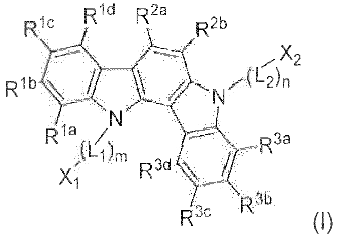 wherein,
R^{1a}, R^{1b}, R^{1c}, R^{1d}, R^{2a}, R^{2b}, R^{3a}, R^{3b}, R^{3c} and R^{3d} are independently of each other a hydrogen atom, a substituted or unsubstituted alkyl or cycloalkyl group having 1 to 20 carbon atoms, a substituted or unsubstituted alkoxy or cycloalkoxy group having 1 to 20 carbon atoms, a substituted or unsubstituted alkenyl group having 2 to 20 carbon atoms, a substituted or unsubstituted alkynyl group having 2 to 20 carbon atoms, a substituted or unsubstituted aryl group having 6 to 30 ring carbon atoms, a substituted or unsubstituted heteroaryl group having 3 to 30 ring carbon atoms,
wherein
at least two adjacent groups R^{1a} and R^{1b}, R^{1b} and R^{1c} or R^{1c} and R^{1d}, R^{2a} and R^{2b}, and/or R^{3a} and R^{3b}, R^{3b} and R^{3c} or R^{3c} and R^{3d} are linked together to form a substituted or unsubstituted ring;
L₁ and L₂ are each independently a direct bond, a substituted or unsubstituted arylene group having 6 to 30 ring carbon atoms, a substituted or unsubstituted heteroarylene group having 3 to 30 ring carbon atoms;
X₁ and X₂ are each independently a substituted or unsubstituted aryl group having 6 to 30 ring carbon atoms, a substituted or unsubstituted O- or S-containing heteroaryl group having 4 to 30 ring carbon atoms;
m and n are independently of each other 1 or 2,
wherein at least one of X₁ and X₂ represents a substituted or unsubstituted fluoranthene unit.

2. The polycyclic compound according to claim 1, wherein two adjacent groups R^{1a} and R^{1b}, R^{1b} and R^{1c}, R^{1c} and R^{1d} or R^{2a} and R^{2b} are linked together to form an unsaturated substituted or unsubstituted six-membered ring.

3. The polycyclic compound according to claim 1 or 2 which is represented by following formulae: 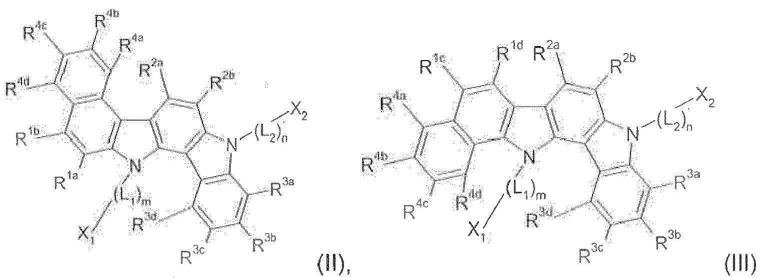 or 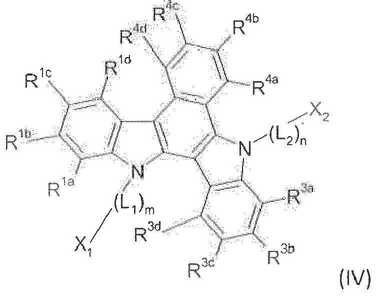 wherein
R^{1a}, R^{1b}, R^{1c}, R^{1d}, R^{2a}, R^{2b}, R^{3a}, R^{3b}, R^{3c}, R^{3d}, R^{4a}, R^{4b}, R^{4c}, R^{4d} are independently of each other a hydrogen atom, a substituted or unsubstituted alkyl or cycloalkyl group having 1 to 20 carbon atoms, a substituted or unsubstituted alkoxy or cycloalkoxy group having 1 to 20 carbon atoms, a substituted or unsubstituted alkenyl group having 2 to 20 carbon atoms, a substituted or unsubstituted alkynyl group having 2 to 20 carbon atoms, a substituted or unsubstituted aryl group having 6 to 30 ring carbon atoms, a substituted or unsubstituted heteroaryl group having 3 to 30 ring carbon atoms
or
two adjacent groups R^{1a} and R^{1b}, R^{1b} and R^{1c}, or R^{1c} and R^{1d}, R^{2a} and R^{2b}, R^{3a} and R^{3b}, R^{3b} and R^{3c} or R^{3c} and R^{3d} and/or R^{4a} and R^{4b}, R^{4b} and R^{4c} or R^{4c} and R^{4d} may be linked together to form an unsaturated six-membered ring.

4. The polycyclic compound according to any one of claims 1 to 3 which is represented by following formulae:

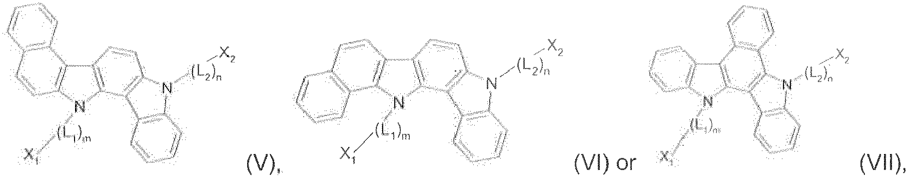

5. The polycyclic compound according to any one of claims 1 to 4, wherein L₁ and L₂ each represent a direct bond or an unsubstituted arylene group having 6 to 10 ring carbon atoms.

6. The polycyclic compound according to claim 5, wherein L₁ and L₂ each represent a direct bond.

7. The polycyclic compound according to any one of claims 1 to 6, wherein either X₁ or X₂ is selected from the following fluoranthene units 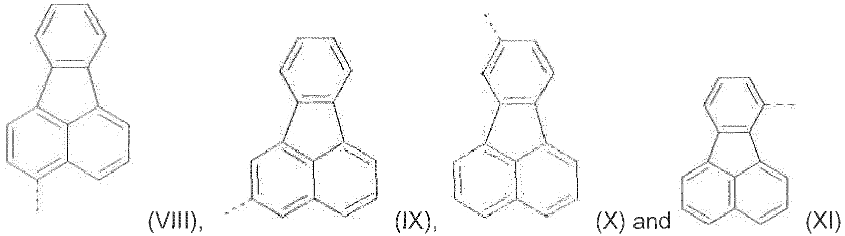 wherein the dotted line represents a bonding site.

8. The polycyclic compound according to any one of claims 1 to 7, wherein either X₁ or X₂ is an aryl group having 6 to 30 ring carbon atoms.

9. A process for the preparation of a compound according to the general formula (I) as defined in any one of claims 1 to 8, at least comprising step (A):
A Coupling a compound of formula (Ia) respectively (Ib) 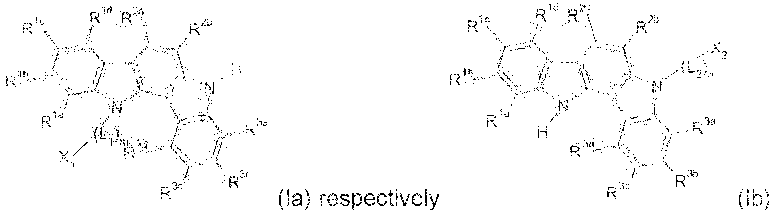 with a compound of formula 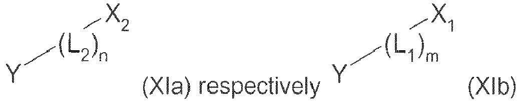 in the presence of a Pd(0) or a Pd(II) salt as a catalyst,
wherein
Y is a halide selected from the group consisting of I, F, Cl and Br, or a pseudohalide selected from the group consisting of mesylate, triflate, tosylate and nonaflate.

10. An electronic device comprising at least one compound as defined in any one of claims 1 to 8.

11. The electronic device according to claim 10, comprising a cathode, an anode, and a plurality of organic thin film layers provided between the cathode and the anode, the organic thin film layers comprising an emitting layer comprising the at least one compound of general formula (I).

12. The electronic device according to claim 10 or 11, wherein the emitting layer comprises a phosphorescent material, which is an ortho-metallated complex comprising a metal atom selected from iridium (Ir), osmium (Os) and platinum (Pt).

13. An electronic equipment comprising the organic electroluminescence device according to any of claims 10 to 12.

14. An emitting layer comprising at least one compound of general formula (I) as defined in any of claims 1 to 8.

15. Use of a compound according to general formula (I) as defined in any of claims 1 to 8 in an electronic device.
